# EUROPEAN PATENT SPECIFICATION

(11) **EP 4 297 671 B1**
(45) Date of publication and mention of the grant of the patent: **03.09.2025**
(21) Application number: 22712296.7
(22) Date of filing: 23.02.2022
(51) Int. Cl.: A61B 17/221, A61B 17/22, A61B 17/00, A61F 2/915

(54) **AN EXPANDABLE CLOT MOBILIZER DEVICE FOR EXTRACTION OF AN OCCLUSION FROM A BLOOD VESSEL**
EXPANDIERBARE VORRICHTUNG ZUR MOBILISIERUNG VON BLUTGERINNSELN ZUR EXTRAKTION EINES VERSCHLUSSES AUS EINEM BLUTGEFÄSS
DISPOSITIF EXTENSIBLE POUR LE DEPLACEMENT ET L'EXTRACTION D'UNE OCCLUSION D'UN VAISSEAU SANGUIN

(30) Priority: 26.02.2021 EP 21382165; 14.07.2021 WO PCT/EP2021/069623; 21.12.2021 EP 21383179
(43) Date of publication of application: 03.01.2024
(73) Proprietor: Anaconda BioMed, SL, 08192 Sant Quirze del Vallès Barcelona (ES)
(72) Inventor: GARCIA SABIDO, Daniel, 08720 Vilafranca del Penedès (ES); FERNÁNDEZ SÁNCHEZ, David, 08206 Sabadell (ES); LIZARAZU GONZALEZ, Ane, 08013 Barcelona (ES); GALVE MURILLO, Iñaki, 08012 Barcelona (ES); ARAD HADAR, Ofir, 08197 Sant Cugat del Vallès (ES); NISSL, Thomas J.W., 37318 Mackenrode (DE); JARA MUNS, Francesc, 08029 Barcelona (ES); PACE, Alizée, 1295 Mies, Vaud (CH)
(74) Representative: Torner, Juncosa I Associats, SL
(86) International application number: PCT/EP2022/054503
(87) International publication number: WO 2022/180084

(56) References cited:
- WO-A1-2019/055311
- WO-A1-2021/016213
- US-A1- 2006 058 836
- US-A1- 2017 020 555
- US-A1- 2019 239 907
- US-B2- 9 700 331

## Description

### Technical Field

The present invention is directed, in general, to the field of intravascular medical devices and in particular, to a clot mobilizer device for extraction of an occlusion from a blood vessel.

### Background of the Invention

Clot mobilizers are self-expanding devices utilized to capture and remove a blood clot or thrombus from a blood vessel. The clot mobilizer is firstly introduced into the blood vessel with a catheter, and then is inserted into the thrombus and deployed. Upon deployment, the clot mobilizer expands to capture the thrombus. The clot mobilizer is removed from the blood vessel allowing blood flow restoration.

Two known clot mobilizers are the Solitaire^{™} X revascularization device manufactured by Medtronic (hereinafter Solitaire) and the Trevo^{®} XP ProVue Retriever manufactured by Concentric Medical (hereinafter Trevo). Both devices are designed for use in the flow restoration of patients with ischemic stroke due to large intracranial vessel occlusion. The stent-like design of the devices allows immediate clot retrieval and blood flow restoration. Similar to the clot mobilizer of present invention, both devices comprise an elongated body with a plurality of interconnected struts in an expanded configuration define a tubular-shaped section. Unlike the clot mobilizer described herein, however, when expanded these prior devices have a pattern defined by almond-shaped cells in the case of the Solitaire, and by large-sized cells in the case of the Trevo. In addition, the Solitaire has an open structure with overlapping longitudinal edges, unlike the closed tubular structure of the clot mobilizer of this invention.

Besides, patent US10426644 provides a medical device for blood flow restoration and/or for use as an implantable member in a human vessel and includes a self-expanding member, a guidewire, and a connection mechanism. The self-expanding member includes a plurality of cells and filaments having specific ranges of thicknesses, widths, and heights. The self-expanding member can take on a volume-reduced coiled form with overlapped edges, and can generate radial forces against a vessel wall and/or thrombus when deployed and expanded.

Likewise, patent US8940003 discloses methods for restoring blood flow in occluded blood vessels using an apparatus having a self-expandable distal segment that is pre-formed to assume a superimposed structure in an unconstrained condition but can be made to take on a volume-reduced form making it possible to introduce it with a microcatheter and a push wire arranged at the proximal end, with the distal segment in its superimposed structure assuming the form of a longitudinally open tube and having a mesh structure of interconnected strings or filaments or struts. The distal segment can have a tapering structure at its proximal end where the strings or filaments or struts converge at a connection point. The distal segment consists of a mesh or honeycomb structure that comprises a multitude of filaments interconnected by a laser welding technique.

On the other hand, patent US10292804 provides an embolectomy device with a clot engaging structure comprising a plurality of interconnected struts forming an open cell structure having an inner lumen. The clot engaging structure is configured to expand from a radially constrained configuration to a radially expanded configuration for deployment within a vessel or duct of a patient. The clot engaging structure can comprise support structure positioned within its inner lumen, the support structure comprising a plurality of connectors connected to respective struts of the clot engaging structure.

Likewise, patent US9700331 discloses a device with a self-expandable member having a proximal end portion and a main body portion. The self-expandable member is movable from a first delivery unexpanded position to a second radially expanded position for deployment within a vessel or duct of a patient. The expandable member includes a plurality of diagonally disposed cell structures. The expandable member has a proximal antenna and a cylindrical main body portion. The cell structures in the cylindrical main body portion comprise proximal and distal flexure elements that are interconnected by a pair of diagonally extending and circumferentially spaced-apart struts having one or more wires or ribbons wound thereabout so as to enhance the average deflection stiffness.

US 2017/020555 A1 describes a system for providing progressive therapy for clot management. The system comprises a microcatheter, a first expandable tip assembly comprising a first elongate member and a first self-expanding scaffold. The first self-expanding scaffold comprises open cells formed by a pattern of struts and bridges. The cells can have a cell size configured to hinder, inhibit, or reduce penetration, or protrusion, of clot material within the scaffold, thereby increasing an amount of blood flow through the scaffold. The system may comprise a second expandable tip assembly comprising a second elongate member and a second self-expanding scaffold. The second self-expanding scaffold comprises open cells formed by a pattern of struts and bridges. The cells of the self-expanding scaffold can have a cell size larger than the cell size of the first self-expanding scaffold. The larger cell size can be configured to enhance penetration, or protrusion, of clot material within scaffold to facilitate capture of the thrombus.

US 2006/058836 A1 discloses a thromboembolic removal system that includes a guide and occlusion catheter, a delivery and aspiration catheter, a thromboembolic disrupter or separator, and an aspiration pump. The thromboembolic removal system advantageously provides the ability to remove a thromboembolism from a cerebral artery within a patient.

WO 2021/016213 A1 discloses an aspiration catheter for removing clot from a blood vessel. It includes a catheter body having a scaffold extending distally from a distal end of the body. An aspiration lumen runs from the distal end to a proximal end of the body, and a central clot-receiving passage in the scaffold is contiguous with the aspiration lumen of the catheter body. A vacuum-resistant membrane covers the scaffold and establishes a clot aspiration path from a distal end of the scaffold to a proximal end of the aspiration lumen, in the catheter body so that applying a vacuum to the proximal end of the aspiration lumen can draw clot into the central clot-receiving passage. The scaffold may have a comical configuration, a cylindrical configuration, or a combination thereof, and at least a distal portion of the scaffold is radially expandable from a delivery configuration to an extraction configuration.

There are at least three shortcomings of some prior clot mobilizers. First, while blood vessels requiring clot removal can present a diameter within a broad range of diameters, many prior clot mobilizers do not expand to provide an appropriate radially-outward force (i.e., by providing enough radial force to engage and remove the clot without exerting a force great enough to damage the blood vessel) over a broad enough range of blood vessel diameters. Prior clot mobilizers are therefore designed in a variety of sizes so that the user can select the proper size for each intervention.

Second, clot mobilizers must often be advanced through a tortuous vasculature in their compressed configurations to reach the clot location. Some prior clot mobilizer designs have not successfully balanced flexibility in their compressed configurations with the appropriate radially outward force in their expanded configurations.

Finally, since it is advanced through microcatheter to the clot location, a clot mobilizer must be able to be pushed without buckling or kinking. Some prior clot mobilizer designs have not successfully combined pushability with flexibility and adequate radial force.

In particular, the known devices/apparatus do not comprise a working portion having a plurality of crowns of cells, each cell comprising an open area bordered by two proximal cell struts, two distal cell struts, and two middle cell struts, the middle cell struts being adapted and configured to be more flexible than the distal and proximal cell struts to which they extend. The known devices/apparatus neither comprise middle cell struts with a width less than a width of the distal and proximal cell struts to which they extend. Furthermore, regarding the Solitaire, this device is formed by a layer comprising two overlapping edges and thus, not forming a closed tube or closed structure. Regarding the Trevo, this device is formed by a tube comprising a plurality of irregular cells bordered by struts. Said struts have one or more wires or ribbons wound thereabout.

### Description of the Invention and additional aspects of the disclosure

To that end, the present invention provides an expandable clot mobilizer device for extraction of an occlusion from a blood vessel according to claim 1. Embodiments are defined in the dependent claims. The clot mobilizer device comprises a working portion having a plurality of crowns (or rows) of cells. Each cell of the working portion includes an open area bordered by two proximal cell struts, two distal cell struts, and two middle cell struts, where: the proximal cell struts each extends distally from a common proximal end to a proximal end of a respective one of the two middle cell struts, the distal cell struts each extends proximally from a common distal end to a distal end of a respective one of the two middle cell struts, each middle cell strut in each crown borders two adjacent cells in that crown. Each of the middle cell struts is adapted and configured to be more flexible than the distal cell strut and proximal cell strut to which it extends. Thus, the cells comprising middle cell struts are configured to transfer a desired flexibility to the working portion. Likewise, the working portion struts are configured to transfer a desired flexibility to the clot mobilizer to be navigated through the vasculature (e.g., the neuroanatomy).

According to the invention, the expandable clot mobilizer device also comprises a tapered portion extending proximally from a proximal end of the working portion, the tapered portion also having a plurality of tapered portion struts. The tapered portion has a smaller diameter at a proximal end than a diameter of the working portion in an expanded configuration. The working portion comprises at least one crown of intermediate cells. Each intermediate cell comprises an open area bordered by two proximal cell struts intersecting with two distal cell struts.

In some embodiments, the tapered portion struts can also have a width greater than a width of the working portion struts, e.g. the distal cell struts, the proximal cell struts and/or the middle cell struts. The tapered portion struts are configured to augment the radial outward force provided by the working portion and to provide pushability to the clot mobilizer to be navigated through the vasculature (e.g., the neuroanatomy) while minimizing buckling and kinking.

Advantageously, in the proposed clot mobilizer, the outward radial forces are maintained for a broad range of vessels (as is seen in Example 3), and better pushability of the clot mobilizer device is achieved. This behavior allows lower radial forces in small diameters and larger radial forces in large diameters without leaving a safety window. Remarkably, due to the fact of having less radial force in small diameters, the risk of damaging the vessel is reduced, in comparison with the Trevo or Solitaire devices (or other known devices for removing clots). Moreover, the proposed clot mobilizer device can be used in vessels of different diameters (between 1.5-4 mm).

According to the embodiments of the invention, the clot mobilizer device is configured to have a compressed (or retracted) configuration (or position or state) with a compressed diameter, and an expanded configuration (or position or state) with an expanded diameter. The clot mobilizer device has the compressed configuration for example when it is located within a lumen of a narrow catheter or a lumen of a narrow vessel with a small diameter, and has the expanded configuration for example when it is located within a lumen of a vessel with large diameter. The clot mobilizer device is configured to self-expand from the compressed position (with a compressed diameter or first diameter) to an expanded position (with an expanded diameter or second diameter). In other words, the clot mobilizer device is self-expandable from the compressed configuration to the expanded configuration. Additionally, the clot mobilizer device is configured to adapt its shape to the surrounding blood vessel, thus the clot mobilizer device can have a first diameter in a narrow vessel with a small diameter and a second diameter in a wide vessel with a large diameter.

Alternatively, in another aspect, the invention provides an expandable clot mobilizer device for extraction of an occlusion from a blood vessel. The clot mobilizer device comprises a working portion having a plurality of crowns of cells. Each cell of the working portion includes an open area bordered by two proximal cell struts, two distal cell struts, and two middle cell struts, where: the proximal cell struts each extends distally from a common proximal end to a proximal end of a respective one of the two middle cell struts, the distal cell struts each extends proximally from a common distal end to a distal end of a respective one of the two middle cell struts, each middle cell strut in each crown borders two adjacent cells in that crown. Each of the middle cell struts is adapted and configured to be more flexible than the distal cell strut and proximal cell strut to which it extends. The working portion is configured to expand from a compressed configuration with a first diameter of less than 1.5 mm to an expanded configuration with a second diameter of at least 3.0 mm and to exert an outward radial force between 0.5 N and 3 N at every diameter between and including the first diameter and the second diameter.

In some embodiments, the working portion is configured to exert an outward radial force between 0.5 N and 3 N in a diameter (first diameter) between 1.5 mm and 3.5 mm. In other embodiments, the working portion is configured to exert an outward radial force between 1.0 N and 2.0 N in a diameter (second diameter) between 2.0 mm and 3.0 mm.

In an embodiment, the working portion is configured to exert an outward radial force between 1.4 N and 3.0 N in a first diameter around 1.5 mm; and wherein the working portion is configured to exert an outward radial force between 0.5 N and 1.4 N in a second diameter around 3.5 mm.

In an embodiment, the working portion is configured to exert an outward radial force between 1 N and 2 N in a diameter around 2.0 mm. In an embodiment, the working portion is configured to exert an outward radial force between 1 N and 2 N in a diameter around 3.0 mm.

According to this aspect, the clot mobilizer device can also comprise a tapered portion extending proximally from a proximal end of the working portion, and comprising a plurality of tapered portion struts; the tapered portion having a smaller diameter at a proximal end than a diameter of the working portion in an expanded configuration. Likewise, in an embodiment, the tapered portion struts can also have a width greater than a width of the working portion struts, e.g., the distal cell struts, the proximal cell struts and/or the middle cell struts.

Alternatively, in another aspect, the invention provides an expandable clot mobilizer device for extraction of an occlusion from a blood vessel. The clot mobilizer device comprises a working portion having a plurality of crowns (or rows) of cells. Each cell of the working portion includes an open area bordered by two proximal cell struts, two distal cell struts, and two middle cell struts, where: the proximal cell struts each extends distally from a common proximal end to a proximal end of a respective one of the two middle cell struts, the distal cell struts each extends proximally from a common distal end to a distal end of a respective one of the two middle cell struts, each middle cell strut in each crown borders two adjacent cells in that crown. The middle cell struts bordering each cell have a width less than a width of the distal cell struts bordering that cell and less than a width of the proximal cell struts bordering that cell.

In an embodiment, the tapered portion struts can also have a width greater than a width of the working portion struts, e.g. the distal cell struts, the proximal cell struts and/or the middle cell struts.

In an embodiment, the plurality of crowns of cells in the working portion of the clot mobilizer device define a tubular-shaped section forming a cylindrically closed structure. In other words, the working portion defines a tubular-shaped section forming a cylindrically closed structure. To that effect, the proposed clot mobilizer device can be manufactured by producing specified cuts either on a tube or on a wire. In another embodiment, the clot mobilizer device comprises a pusher (or pusher wire) manufactured by producing specified cuts on said wire. A pusher is configured to maneuver the clot mobilizer (e.g. advance the device distally, withdraw the device proximally, and rotate the device about a longitudinal axis from a proximal end of the pusher).

In an embodiment, the clot mobilizer is manufactured by providing a tube; having a longitudinal axis therethrough, providing a stationary source of laser radiation, generating a beam of laser radiation using the source of laser radiation, and cutting a desired pattern into the tube by scanning the beam over a desired region of the tube. In another embodiment, the clot mobilizer is manufactured by providing a wire; having a longitudinal axis therethrough, producing predetermined cuts of the cross section of the wire by means of an ultrashort pulse laser in order to produce a predetermined shape of the stent. A suitable manufacturing method is described e.g., in US10434605B2.

In an embodiment, the working portion is configured to have a compressed configuration with a first diameter of less than 1.5 mm and to exert an outward radial force between 1.4 N and 3 N. For example, 1.4 N, 1.5 N, 2 N or 2.5 N when the first diameter is around 1.5 mm.

In an embodiment, the working portion is configured to have an expanded configuration with a second diameter of at least 3.0 mm and to exert an outward radial force between 0.5 N and 1.5 N. For example, 0.6 N, 1 N, 1.4 N or 1.5 N when the second diameter is around 3.0 mm. In another embodiment, the working portion is configured to have an expanded configuration with a second diameter of at least 3.5 mm and to exert an outward radial force between 0.5 N and 1.5 N. For example, 0.5 N, 1 N, 1.15 N or 1.4 N when the second diameter is around 3.5 mm.

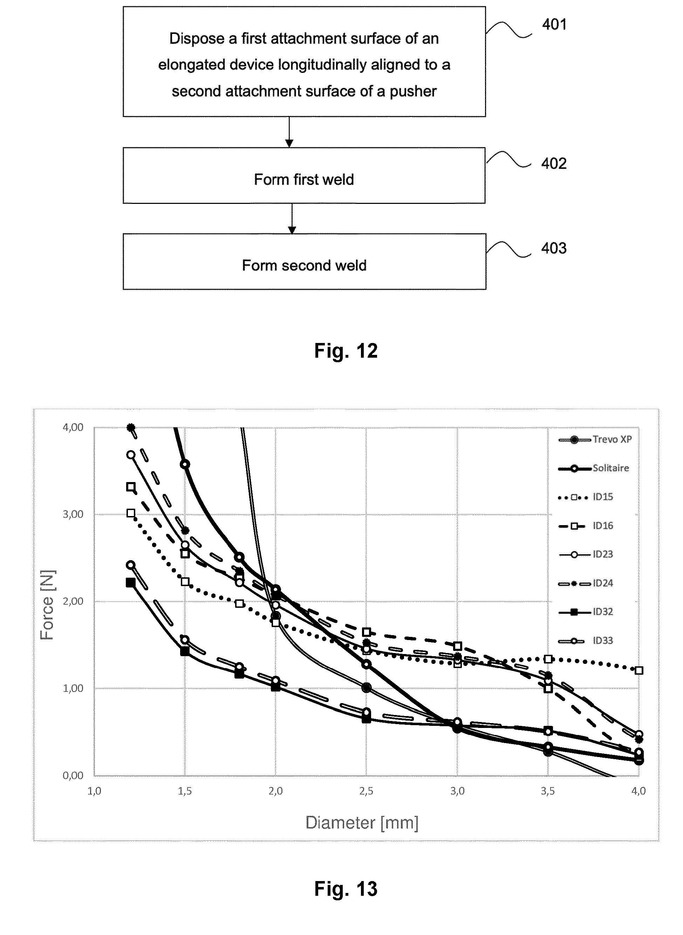

In an embodiment, the working portion is configured to exert an outward radial force in the range between 0.5 N and 3 N at every diameter between and including a first diameter of 1.5 mm (in a compressed configuration) and a second diameter of 3.5 mm (in an expanded configuration), resulting in a flat curve behavior (as it can be seen in Fig. 13), allowing lower radial forces in small diameters and larger radial forces in large diameters, without leaving a safety window. Consequently, the outward radial forces are maintained for a broad range of vessels with different diameters and the risk of damaging the vessel is reduced. In a particular embodiment, the working portion is configured to exert an outward radial force in the range, inside a safety window, between 1 N and 2.1 N at every diameter between and including a first diameter of 1.5 mm and a second diameter of 3.5 mm, e.g., a first diameter of 2 mm and a second diameter of 3.5 mm or e.g. a first diameter of 2 mm and a second diameter of 3.0 mm.

In an embodiment, each middle cell strut in the working portion can comprise at least a hinge portion. Each hinge portion is disposed between the proximal and distal ends of the middle cell strut. In an embodiment, each middle cell strut in the working portion can comprise a hinge portion. In an embodiment, each middle cell strut in the working portion can comprise a hinge portion disposed between its proximal and distal ends. In another embodiment, each middle cell strut in the working portion can comprise two hinge portions.

In an embodiment, the hinge portion can comprise at least a bend. In an embodiment, the hinge portion can comprise a bend. In some embodiments, the bend is disposed in a central portion of the middle cell strut. In other embodiments, the bend is disposed closer to one end of the middle cell strut than to another end of the middle cell strut.

In an embodiment, the hinge portion can comprise two bends.

In some embodiments, the hinge portion can comprise a section of increased curvature.

In some embodiments, the hinge portion and middle cell strut are two parts of a single structure. The hinge portion is therefore integral with the middle cell strut.

In some embodiments, the middle cell struts bordering each cell in the working portion have a width less than a width of the distal cell struts bordering that cell. In some embodiments, the middle cell struts bordering each cell in the working portion have a width less than a width of the proximal cell struts bordering that cell. In some embodiments, the middle cell struts bordering each cell have a width less than a width of the distal cell struts bordering that cell and less than a width of the proximal cell struts bordering that cell.

In some embodiments, in each crown of cells in the working portion, the distal cell struts are disposed in a distal cell strut ring at equal distal cell strut positions along a longitudinal axis of the clot mobilizer device. In other embodiments, in each crown of cells in the working portion, the proximal cell struts are disposed in a proximal cell strut ring at equal proximal cell strut positions along the longitudinal axis of the clot mobilizer device. In other embodiments, in each crown of cells, the common proximal ends of the distal cell strut ring (i.e., the distal ends of the middle cell struts) are circumferentially offset from the common distal ends of the proximal cell strut ring (i.e., the proximal ends of the middle cell struts).

In an embodiment, the common proximal ends of the distal cell strut ring of each crown of cells of the plurality of crowns of cells are circumferentially offset from the common distal ends of the proximal cell strut ring of that crown of cells in one circumferential direction.

In an embodiment, the common proximal ends of the distal cell strut ring of a first crown of cells of the plurality of crowns of cells are offset from the common distal ends of the proximal cell strut ring of the first crown of cells in a first circumferential direction and the common proximal ends of the distal cell strut ring of a second crown of cells of the plurality of crowns of cells are offset from the common distal ends of the proximal cell strut ring of the second crown of cells in a second circumferential direction opposite to the first circumferential direction.

In some embodiments, the distal cell strut ring of a specific crown of cells of the plurality of crowns of cells can correspond to the proximal cell strut ring of the corresponding adjacent crown of cells of the plurality of crowns of cells. In some embodiments, the distal cell strut ring of a specific crown of cells of the plurality of crowns of cells can comprise the proximal cell struts of cells in an adjacent crown of cells.

In some embodiments, the proximal cell strut ring of a specific crown of cells of the plurality of crowns of cells can correspond to the distal cell strut ring of the corresponding adjacent crown of cells of the plurality of crowns of cells. In some embodiments, the proximal cell strut ring of a specific crown of cells of the plurality of crowns of cells can comprise the distal cell struts of cells in an adjacent crown of cells.

In some embodiments, the middle cell struts of a second crown of cells of the plurality of crowns of cells extend distally from common distal ends of distal cell struts of a first crown of cells of the plurality of crowns of cells to common proximal ends of proximal cell struts of a third crown of cells of the plurality of crowns of cells, and the middle cell struts of the third crown of cells extend distally from common distal ends of distal cell struts of the second crown of cells to common proximal ends of proximal cell struts of a fourth crown of cells of the plurality of crowns of cells.

In some embodiments, the distal cell struts of each intermediate cell in the at least one crown of intermediate cells can comprise the proximal cell struts of cells in an adjacent crown of cells. In some embodiments, the proximal cell struts of each intermediate cell in the at least one crown of intermediate cells can comprise the distal cell struts of cells in an adjacent crown of cells. The crowns of cells adjacent to the at least one crown of intermediate cells can comprise middle cell struts adapted and configured to be more flexible than the distal cell strut and proximal cell strut to which each middle cell strut extends. Therefore, the cells comprising middle cell struts are configured to transfer a desired flexibility to the working portion. And the intermediate cells are configured to transfer strength to the working portion, thus avoiding kinks (or breaks).

In some embodiments, the cells in the working portion have a substantially almond shape. In other embodiments, the cells in the working portion have a substantially oval shape.

In some embodiments, the cells in the working portion have an irregular almond shape. In other embodiments, the cells in the working portion have an irregular oval shape.

For purposes of this disclosure, the shape of the cells is "irregular" when the cells comprise middle cell struts with bends or increased curvature which alter the predefined shape, such as almond or oval.

In some embodiments, the number of crowns of cells depends on the desired length of the working portion. In some embodiments, the working portion can have at least four crowns of cells.

In some embodiments, the working portion can have between four and twelve crowns of cells. Particularly, the working portion can have between six and ten crowns of cells. More in particular, the working portion can have seven, eight or nine crowns of cells.

In some embodiments, each crown of cells in the working portion can have at least three cells. In some embodiments, each crown of cells in the working portion can have at least four cells. In some embodiments, each crown of cells in the working portion can have between four and ten cells, particularly, between four and eight cells. In an embodiment, each crown of cells in the working portion can have four or six cells.

In an embodiment, each crown of cells in the working portion can have four cells. In another embodiment, each crown of cells in the working portion can have six cells.

In an embodiment, the working portion comprises at least four crowns of cells and each crown of cells comprises at least four cells. In another embodiment, the working portion comprises eight crowns of cells and each crown of cells comprises four cells. In another embodiment, the working portion comprises seven crowns of cells and each crown of cells comprises four cells. In another embodiment, the working portion comprises nine crowns of cells and each crown of cells comprises six cells.

In some embodiments, the distal cell struts in the working portion each can have a width greater than or equal to 0.08 mm and less than or equal to 0.13 mm. In other embodiments, the distal cell struts in the working portion each can have a width greater than or equal to 0.10 mm and less than or equal to 0.125 mm. Particularly, the distal cell struts in the working portion each can have a width of 0.10 mm, 0.12 mm, 0.124 mm or 0.125 mm.

In some embodiments, the proximal cell struts in the working portion each have a width greater than or equal to 0.08 mm and less than or equal to 0.13 mm. In other embodiments, the proximal cell struts in the working portion each can have a width greater than or equal to 0.10 mm and less than or equal to 0.125 mm. Particularly, the proximal cell struts in the working portion each can have a width of 0.10 mm, 0.12 mm, 0.124 mm or 0.125 mm.

In some embodiments, the middle cell struts in the working portion each can have a width greater than or equal to 0.05 mm and less than or equal to 0.13 mm. The middle cell struts in the working portion each can have a width greater than or equal to 0.08 mm and less than or equal to 0.125 mm. The middle cell struts in the working portion each can have a width greater than or equal to 0.08 mm and less than or equal to 0.10 mm. The middle cell struts in the working portion each can have a width of 0.055 mm, 0.075 mm 0.08 mm, 0.09 mm 0.10 mm or 0.125 mm.

In some embodiments, the distal cell struts each have a width greater than or equal to 0.10 mm and less than or equal to 0.145 mm, the proximal cell struts each have a width greater than or equal to 0.10 mm and less than or equal to 0.145 mm, and the middle cell struts each have a width greater than or equal to 0.05 mm and less than or equal to 0.10 mm.

In an embodiment, the distal cell struts and the proximal cell struts can have substantially the same width.

In some embodiments, the working portion can have a length between 30 mm and 50 mm, particularly between 38 mm and 45 mm, e.g., 40 mm, 41.8 mm, 42 mm or 42.5 mm.

In some embodiments, the working portion in the expanded configuration can have a length between 30 mm and 50 mm, particularly between 40 mm and 45 mm, e.g., 40 mm, 41.8 mm, 42 mm or 42.5 mm.

In some embodiments, the working portion in the compressed configuration can have a length between 30 mm and 50 mm, particularly between 38 mm and 45 mm, e.g., 40 mm, 41.8 mm, 42.5 mm or 45 mm.

In some embodiments, the working portion in the compressed configuration can have a length between 40 mm and 45 mm, e.g., 42 or 42.5 mm, and the working portion in the expanded configuration can have a length between 40 mm and 45 mm, e.g., 40 mm.

In some embodiments, the tapered portion can have a length between 5 mm and 20 mm, between 5 mm and 15 mm, or between 5 mm and 12 mm, e.g., a length of 5 mm or 9 mm.

In some embodiments, the tapered portion in the expanded configuration can have a length between 5 mm and 20 mm, between 5 mm and 15 mm, or between 5 mm and 12 mm, e.g., a length of 5 mm or 9 mm.

In some embodiments, the tapered portion in the compressed configuration can have a length between 5 mm and 20 mm, between 5 mm and 15 mm, or between 5 mm and 12 mm, e.g., a length of 5 mm or 9 mm.

In an embodiment, the tapered portion struts have a thickness equal to a thickness of the working portion struts.

In an embodiment, at least some of the plurality of tapered portion struts border a plurality of tapered portion cells. In embodiments, the number of tapered portion struts can be 4, 5, 9, 14, or any other suitable number. The number of tapered portion cells can be 3, 5, 6, or any other suitable number.

In an embodiment, at least some of the plurality of tapered portion struts converge from a proximal end of the working portion to a distal end of a proximal connection portion.

In an embodiment, some of the plurality of tapered portion struts converge proximally to the distal end of the proximal connection portion.

In some embodiments, the tapered portion struts are wider than the working portion struts. In some embodiments, the tapered portion struts are wider than the distal cell struts and the proximal cell struts. In other embodiments, the tapered portion struts are wider than the middle cell struts.

In some embodiments, the tapered portion struts can have a width equal to or greater than 0.10 mm and less than or equal to 0.14 mm. In other embodiments, the tapered portion struts can have a width of 0.10 mm, 0.11 mm, 0.12 mm, 0.13 mm, or 0.14 mm. In some embodiments, the tapered portion struts can each have a uniform width, and in other embodiments one or more of the tapered portion struts can have widths that vary in different sections.

In an embodiment, the clot mobilizer device further comprises a pusher extending proximally from the proximal connection portion (or first connection portion).

In an embodiment, the proximal connection portion acts as a pusher connector.

In an embodiment, the proximal connection portion can have two proximally extending arms. In an embodiment, the proximal connection portion acts as pusher connector to connect the pusher to the clot mobilizer device through the two extending arms. In an embodiment, the proximal connection portion can have a single proximally extending arm. In an embodiment, the proximal connection portion acts as pusher connector to connect the pusher to the clot mobilizer device through the single extending arm.

Embodiments of the present invention provide a clot mobilizer device with an improved attachment of their elements. The clot mobilizer device can be advanced distally and withdrawn proximally from a proximal end and rotated about a longitudinal axis from the proximal end. The clot mobilizer device comprises an elongated device comprising a working portion and a tapered portion. The elongated device comprises a first connection portion (or a proximal connection portion) extending proximally from the tapered portion. The first connection portion has a first attachment surface. The clot mobilizer device also comprises a pusher comprising a second connection portion extending distally, the second connection portion having a second attachment surface facing and extending longitudinally aligned to the first attachment surface to define an overlapping portion and to define first and second seams extending longitudinally along first and second lateral extents of the overlapping portion. The device also comprises a first weld attaching the first and second connection portions, extending from the first seam toward the second seam (i.e. laterally); and a second weld attaching the first and second connection portions, extending from the second seam toward the first seam.

In an embodiment, the first (or proximal) connection portion can have different forms such as V-shape, U-shape, needle, needle with radiopaque marker, among others.

In an embodiment, a cross-sectional shape of the first connection portion perpendicular to the longitudinal axis is a section of an annulus. In another embodiment, a cross-sectional shape of the second connection portion perpendicular to the longitudinal axis is a circle.

In an embodiment, the first weld comprises a plurality of welding points along the first seam. In another embodiment, the second weld comprises a plurality of welding points along the second seam. In some embodiments, the plurality of welding points of the first and second welds are successive, i.e., the points are aligned.

In some embodiments, the first weld can further comprise a plurality of consecutive welding points. In some embodiments, the second weld can further comprise a plurality of consecutive welding points. Thus, a welding seam is provided on top of the welding points previously applied.

In an embodiment, the first weld and the second weld each extend along an entire length of the overlapping portion.

In an embodiment, a glue is applied over the first and second weld.

In an embodiment, a ratio of a cross-sectional area of the first connection portion perpendicular to the longitudinal axis to a corresponding cross-sectional area of the second connection portion perpendicular to the longitudinal axis is in a range of 1:4 to 2:1. In an embodiment, a radius of the second attachment surface is smaller than a radius of the first attachment surface.

In an embodiment, the clot mobilizer device also includes a jacket extending around at least part of the overlapping portion. The jacket can extend proximally from the overlapping portion around at least part of the pusher.

Additionally, the clot mobilizer device can also include a radiopaque element disposed at a proximal end of the overlapping portion. In an embodiment, the radiopaque element comprises a coil extending around at least part of the pusher.

In an embodiment, the clot mobilizer device also comprises a jacket extending around the radiopaque element. The jacket can extend around at least part of the overlapping portion. In some embodiments, the jackets can comprise a suitable shrinkable material, for example a shrinkable plastic such as a thermoplastic material.

The surgical methods described below are not explicitly recited by the wording of the claims, but are considered as useful for understanding the invention.

Embodiments of the present disclosure also provide, according to another aspect, a method for connecting a pusher to an elongated device (comprising a working portion, and a tapered portion) to enable the pusher to maneuver the elongated device (e.g. advance the device distally, withdraw the device proximally, and rotate the device about a longitudinal axis from a proximal end of the pusher). The elongated device comprises a first connection portion (or a proximal connection portion) extending proximally from the tapered portion. The proximal connection portion acts as a pusher connector. The proximal connection portion can comprise one or two extending arms where the pusher is connected. This first connection portion has a first attachment surface. The pusher comprises a second connection portion extending distally. The second connection portion has a second attachment surface. The method comprises disposing the first attachment surface longitudinally aligned to the second attachment surface to form an overlapping portion and to form first and second seams extending parallel to the longitudinal axis along first and second lateral extents of the overlapping portion; forming a first weld extending from the first seam toward the second seam, and forming a second weld extending from the second seam toward the first seam.

In an embodiment, a cross-sectional shape of the first connection portion perpendicular to the longitudinal axis is a section of an annulus. In another embodiment, a cross-sectional shape of the second connection portion perpendicular to the longitudinal axis is a circle.

In an embodiment, the step of forming the first weld comprises forming a plurality of welding points extending from the first seam toward the second seam. In another embodiment, the step of forming the second weld comprises forming a plurality of welding points extending from the second seam toward the first seam. Particularly, the first and second welds are formed by sequentially forming the plurality of welding points (i.e., the welding points are aligned).

In an embodiment, the step of forming the first weld further comprises consecutively forming a plurality of welding points extending from the first seam toward the second seam. In another embodiment, the step of forming the second weld further comprises consecutively forming a plurality of welding points extending from the second seam toward the first seam.

When energy, e.g., laser energy, is applied to form the first and second welds a heat affected zone appears (i.e., accumulation of heat in a specific zone) which can provoke a breakage of the first, the second, or both connection portions. To avoid that breakage, in some embodiments, the step of forming the first weld further comprises forming a first welding point of the plurality of welding points at a distal portion of the first seam and forming each other welding point of the plurality of welding points at a more proximal location along the first seam than a prior formed welding point of the plurality of welding points. In some embodiments, the step of forming the second weld further comprises forming a first welding point of the plurality of welding points at a distal portion of the second seam and forming each other welding point of the plurality of welding points at a more proximal location along the second seam than a prior formed welding point of the plurality of welding points.

In some embodiments, the first weld can extend along an entire length of the first seam and the second weld can extend along an entire length of the second seam.

In an embodiment, the step of forming a first weld comprises forming a first welding point at a proximal end of the first seam and a second welding point between the proximal end and the distal end of the first seam before forming a welding point at any other points along the first seam.

In an embodiment, the step of forming the second weld comprises forming a first welding point at a proximal end of the second seam and a second welding point between the proximal end and the distal end of the second seam before forming a welding point at any other points along the second seam.

In an embodiment, the step of forming the first weld comprises directing energy laterally at the first seam toward the second seam. In an embodiment, the step of forming the second weld comprises directing energy laterally at the second seam toward the first seam.

In an embodiment, the method also comprises the step of adding glue over the first and second welds.

In an embodiment, the method also comprises the step of placing a radiopaque element at a proximal end of the overlapping portion. In an embodiment, the radiopaque element can comprise a coil extending around at least part of the pusher.

In an embodiment, the method also comprises the step of assembling, or placing, the radiopaque element onto a distal end of the pusher and pushing the radiopaque element towards a proximal end of the pusher.

In an embodiment, the method further comprises covering at least part of the overlapping portion with a jacket. In some embodiments, a portion of the pusher proximal to the overlapping portion can also be covered with the jacket.

In an embodiment, the method further comprises covering the radiopaque element with a jacket. In some embodiments, the jacket also extends around at least part of the overlapping portion.

In an embodiment, before inserting the jacket, the method further comprises placing, or pushing back, the radiopaque element to the distal end of the pusher (or at the proximal end of the overlapping portion), without covering the overlapping portion.

In yet another embodiment, the method comprises covering at least part of the overlapping portion with a jacket (or inner jacket), the jacket extending proximally from the overlapping portion around at least part of the pusher; placing a radiopaque element at a proximal end of the overlapping portion, the radiopaque element comprising a coil extending around at least part of the pusher; and covering the radiopaque element with a jacket (or outer jacket), the jacket extending around at least part of the overlapping portion. In an embodiment, before inserting the inner jacket, the method further comprises assembling, or placing, the radiopaque element onto a distal end of the pusher and pushing the radiopaque element towards a proximal end of the pusher.

In another embodiment, before inserting the outer jacket, the method further comprises placing, or pushing back, the radiopaque element to the distal end of the pusher (or at the proximal end of the overlapping portion), without covering the overlapping portion.

In an embodiment, a heat shrinkable material can be also added to the inner and outer jackets. In another embodiment, the inner and outer jackets can comprise a heat shrinkable material, for example a shrinkable plastic such as a thermoplastic.

In an embodiment, a ratio of a cross-sectional area of the first connection portion perpendicular to the longitudinal axis to a corresponding cross-sectional area of the second connection portion perpendicular to the longitudinal axis is in a range of 1:4 to 2:1, particularly 3:4.

In an embodiment, a radius of the second attachment surface is smaller than a radius of the first attachment surface.

In some embodiments, according to the present disclosure, the clot mobilizer device also includes radiopaque markers located at a distal end of the working portion. In other embodiments the radiopaque markers can be also located at other sections of the working portion, e.g. at the middle and/or at the proximal end. The radiopaque markers particularly have different lengths to avoid entanglement between them or other devices. The radiopaque markers can be made of a Platinum Iridium alloy or of Tantalum.

In an embodiment, the clot mobilizer device is self-expandable.

In an embodiment, the clot mobilizer device is made of a metal including Nitinol. In particular, the Nitinol material complies with the ASTM (American Society of Testing and Materials) F2063 (Standard Specification for Wrought Nickel-Titanium Shape Memory Alloys for Medical Devices and Surgical Implants). Nitinol is well known for applications in self-expanding structures. However, other types of metals or even other types of materials can be also used, e.g., cobalt-chromium alloys or iron alloys such as stainless steel or spring steel; also, other materials with shape memory properties can be used, e.g. cooper or magnesium alloys.

In an embodiment, the clot mobilizer also includes an ultrasound transmission member/device to fragment the thrombus into smaller particles by applying ultrasound energy waves. In a particular embodiment, the ultrasound transmission member comprises a sonotrode operating at high frequency (between 15 and 30 kHz, particularly 19.8 kHz or 20 kHz). The vibrational amplitude of the sonotrode in this case is transmitted through the wire of the clot mobilizer. The vibrational amplitude can range between 3 µm (10%) and 7.5 µm (25%), particularly 3 µm (10%) or 6 µm (20%). The duration of the ultrasound transmission can be e.g., 60 seconds or 120 seconds.

In an embodiment, the ultrasound transmission member particularly extends longitudinally through the axis of the clot mobilizer. Moreover, in some embodiments, a sonic connector can be also coupled to a proximal end of the ultrasound transmission member.

In yet other embodiments, the expandable device also comprises a connection portion extending proximally from a proximal end of the working portion, the connection portion being adapted to be connected to a pusher for advancing the device through the vasculature to an expansion site. Moreover, in some embodiments, the pusher can comprise a tube. The tube can be a catheter or a hypotube.

The connection portion comprises at least one crown of cells, each cell comprising an open area bordered by two proximal cell struts, two distal cell struts, and two middle cell struts, the proximal cell struts each extending distally from a common proximal end to a proximal end of a respective one of the two middle cell struts, the distal cell struts each extending proximally from a common distal end to a distal end of a respective one of the two middle cell struts, each middle cell strut bordering two radially adjacent cells, each of the middle cell struts being adapted and configured to be more flexible than the distal cell strut and proximal cell strut to which it extends.

In an embodiment, the working portion also includes a tapered portion at the proximal end of the working portion. The tapered portion can comprise a plurality of tapered portion struts. The tapered portion has an expanded diameter at a proximal end that is smaller than an expanded diameter of the working portion.

In an embodiment, the tapered portion comprises at least one crown of cells of the plurality of crowns of cells.

In an embodiment, the tapered portion has an expanded configuration defining a frustoconical shape with a closed structure.

In an embodiment, the tapered portion further comprises a crown of cells with each cell comprising an open area bordered by two proximal cell struts and two distal cells struts, in each cell the proximal cell struts each extending distally from a common proximal end to proximal ends of the distal cell struts, the distal cell struts each extending distally from their proximal ends to a common distal end.

In an embodiment, the tapered portion converges at a distal end of the connection portion. In an embodiment, the plurality of crowns of cells of the working portion defines a tubular-shaped section forming a cylindrically closed structure. Alternatively or complementarily, in an embodiment, the connection portion comprises a tubular-shaped section forming a cylindrically closed structure.

In an embodiment, the middle cell struts bordering each cell have a width less than a width of the distal cell struts bordering that cell and less than a width of the proximal cell struts bordering that cell.

Another aspect of the disclosure provides a thrombectomy system which comprises the clot mobilizer device of the present invention and a microcatheter configured to be advanced through vasculature of a patient to a thrombus site within a blood vessel and adapted to carry the clot mobilizer to the thrombus site; wherein the clot mobilizer is configured to be movably disposed within the microcatheter in a compressed configuration and at least partially outside the microcatheter in an expanded configuration.

The aspect previously provided can be used for extracting a thrombus from a thrombus site in a blood vessel by following a method comprising the steps of advancing the microcatheter through vasculature toward the thrombus site, wherein advancing the microcatheter comprises advancing a distal end of the microcatheter through the thrombus, the clot mobilizer device being disposed within the microcatheter; advancing the clot mobilizer until reaching the distal end of the microcatheter, moving the microcatheter and the clot mobilizer device with respect to each other to place the clot mobilizer device outside of the microcatheter allowing the clot mobilizer device to expand from the compressed configuration to the expanded configuration. More particularly, the method further comprises the step of self-expanding the clot mobilizer from the compressed configuration to the expanded configuration.

Another aspect of the disclosure provides a method of extracting a clot from a blood vessel of a patient by advancing an expandable clot mobilizer device into the blood vessel in a compressed configuration having a first diameter of less than 1.5 mm, the clot mobilizer device comprising a plurality of crowns of cells in a working portion, each cell comprising an open area bordered by two proximal cell struts, two distal cell struts, and two middle cell struts, the proximal cell struts each extending distally from a common proximal end to a proximal end of a respective one of the two middle cell struts, the distal cell struts each extending proximally from a common distal end to a distal end of a respective one of the two middle cell struts, each middle cell strut in each crown bordering two adjacent cells in that crown, each of the middle cell struts being adapted and configured to be more flexible than the distal cell strut and proximal cell strut to which it extends. The method further comprises expanding the working portion of the clot mobilizer into the clot to the expanded configuration with a second diameter of at least 3.0 mm; and exerting an outward radial force with the working portion between 0.5 N and 3 N at every diameter between and including the first diameter and the second diameter. In some embodiments, the exerting step includes the step of exerting an outward radial force with the working portion between 0.5 N and 1.5 N when the second diameter is around 3.5 mm. In some embodiments, the clot mobilizer device has a tapered portion extending proximally from the working portion. The method further includes the step of expanding the tapered portion to a diameter smaller than a diameter of the working portion in the expanded configuration. In some embodiments, the advancing step can include the step of advancing a pusher extending proximally from a connection portion of the tapered portion.

In another aspect, the disclosure provides a thrombectomy system which comprises the clot mobilizer device of the present invention; a delivery catheter configured to be advanced through vasculature of a patient to a thrombus site within a blood vessel; and an aspiration catheter adapted to apply suction to an expandable aspiration funnel extending from a distal end of the aspiration catheter. The aspiration funnel is configured to be movably disposed within the delivery catheter in a retracted position (in a compressed state) and at least partially outside the delivery catheter in an expanded position. The aspiration funnel comprises a non-permeable covering, a diameter of a distal end of the aspiration funnel being greater in the expanded position than in the retracted position. The aspiration funnel is configured to adapt its shape and length to an inner wall of the blood vessel such that the aspiration funnel reduces (or stops) blood flow through the blood vessel and lengthens as it narrows to retain a thrombus within the aspiration funnel. Additionally, the clot mobilizer device is configured to capture the thrombus and to be at least partially withdrawn with the captured thrombus into the aspiration funnel. The thrombectomy system further comprises a microcatheter adapted to carry the clot mobilizer device to the thrombus site; wherein the clot mobilizer device is configured to be movably disposed within the microcatheter in a retracted configuration, and wherein the microcatheter is configured to be movably disposed within the aspiration catheter and at least partially outside the microcatheter in an expanded configuration.

In an embodiment, the delivery catheter, the aspiration funnel, the microcatheter and the clot mobilizer device are oriented on a same axis and can be coaxially configured and movable to each other.

The aspect previously provided can be used for extracting a thrombus from a thrombus site in a blood vessel by following a method comprising the steps of advancing a delivery catheter through vasculature toward the thrombus site; placing a distal end of the delivery catheter proximal to the thrombus in the blood vessel; advancing the aspiration catheter within the delivery catheter, the aspiration funnel extending distally from the aspiration catheter; expanding the aspiration funnel into contact with inner walls of the blood vessel, thereby reducing (or stopping) blood flow past the aspiration funnel; advancing the clot mobilizer device distally through the aspiration funnel toward the thrombus; deploying the clot mobilizer device to capture the clot; moving the clot mobilizer device and the thrombus proximally toward the aspiration funnel; applying suction through the aspiration catheter to the aspiration funnel to aspirate the thrombus at least partially into the aspiration funnel; and moving the aspiration funnel and the thrombus proximally within the vasculature, the aspiration funnel adapting its shape and length to a surrounding blood vessel by lengthening as it narrows to retain the thrombus within the aspiration funnel.

In a particular embodiment, the method further comprises the steps of advancing a microcatheter within the aspiration catheter, wherein advancing the microcatheter comprises advancing a distal end of the microcatheter through the thrombus, the clot mobilizer device being disposed within the microcatheter; moving the microcatheter and the clot mobilizer device with respect to each other to place the clot mobilizer device outside of the microcatheter; and expanding the clot-capture device allowing the clot mobilizer device to self-expand. More particularly, the method further comprises the step of moving the clot mobilizer device proximally at least partially into the aspiration funnel.

In an embodiment according to an aspect of the present disclosure, the aspiration funnel is self-expandable.

In an embodiment, the aspiration funnel comprises a segment defining a distal end and a proximal end, wherein the segment is formed by a mesh of at least two sets of helicoidal filaments turning respectively in opposite directions and being intertwined; the mesh comprises two distinct tubular sections, a first section and a second section, wherein the second section, adjacent to the first section, provides a reduction of diameter; and, said mesh of the first section has helicoidal filaments with a braiding angle configured to provide outward radial forces higher than in the second section, such that the first section becomes appositioned against the inner wall of the blood vessel.

In an embodiment, the first section of the mesh comprises closed loops at the distal end configured to act as a spring, such that the outward radial forces in a first and second end portions of the first section are higher than in an intermediate portion thereof.

In an embodiment, the second section of the mesh is comprised of two sub-sections, a first sub-section having a shape with a progressive reduction of diameter configured to open and create a space for the thrombus and to reduce (or stop) a proximal blood flow during the removal of the thrombus, and a second sub-section of a tubular uniform diameter configured to provide a connection to the aspiration catheter. In particularly, the shape of the first sub-section is cone-shaped (or funnel-shaped).

In an embodiment, the two sets of helicoidal filaments are adapted to become more longitudinally aligned as the aspiration funnel lengthens and narrows. In particularly, the helicoidal filaments of the mesh are made of a metal, a metal alloy or a composite including Nitinol or Nitinol/Platinum.

In a particular embodiment, the helicoidal filaments comprise a number ranging between 24 and 48, said filaments having a cross section comprised in a range between 40 and 60 µm; and the angle of the helicoidal filaments with regard to a longitudinal axis of the segment is comprised between 50 and 65 degrees for the first section, and between 15 and 50 for the second sub-section.

In a particular embodiment, the first section comprises a length ranging between 4 and 40 millimeters and the second sub-section comprises a length ranging between 1 and 10 millimeters; the first section comprises an outer diameter ranging between 3.5 and 6 millimeters and the second sub-section comprises an outer diameter ranging between 1 and 2 millimeters; and the shape of the first sub-section comprises a generatrix with an angle comprised between 15 and 45 degrees with regard to a longitudinal axis of the segment.

In a particular embodiment, the non-permeable covering of the aspiration funnel comprises a polymer including silicone or polyurethane.

### Brief Description of the Drawings

The previous and other advantages and features will be more fully understood from the following detailed description of embodiments, with reference to the attached figures, which must be considered in an illustrative and non-limiting manner, in which:
Figs. 1A-6B illustrate different examples of the proposed clot mobilizer device for extraction of an occlusion from a blood vessel. Figs. 6A-6B illustrate a clot mobilizer device according to some embodiments of the present invention.
Figs. 7A-7C illustrate different enlarged views of the working and tapered portions of the clot mobilizer device.
Fig. 8A and 8B illustrate other embodiments of the clot mobilizer device adapted to be advanced distally and withdrawn proximally from a proximal end and rotated about a longitudinal axis from the proximal end.
Figs. 9A-D illustrate different designs of the first connection portion of the clot mobilizer device. Fig. 9A: V-shape; Fig. 9B: U-shape; Fig. 9C: needle shape; and Fig. 9D: needle with radiopaque marker.
Fig. 10 schematically illustrates the geometry of the cross-sectional view of the first connection portion and the second connection portion of the clot mobilizer device, according to an embodiment.
Fig. 11 illustrates a more detailed view of a clot mobilizer device adapted to be advanced distally and withdrawn proximally from a proximal end and rotated about a longitudinal axis from the proximal end, according to another embodiment.
Fig. 12 is a flow chart illustrating a method for connecting an elongated device to a pusher to enable the pusher to maneuver the elongated device, according to an embodiment.
Fig. 13 graphically illustrates a comparison (individual radial force vs. diameter) between the proposed clot mobilizer device, Solitaire and Trevo devices.
Figs. 14A and 14B illustrate two other embodiments of the proposed expandable device.
Figs. 15A and 15B are two different 3D views of the 2D embodiments of the expandable device previously shown in Figs. 14A and 14B, respectively.

### Detailed Description of Embodiments

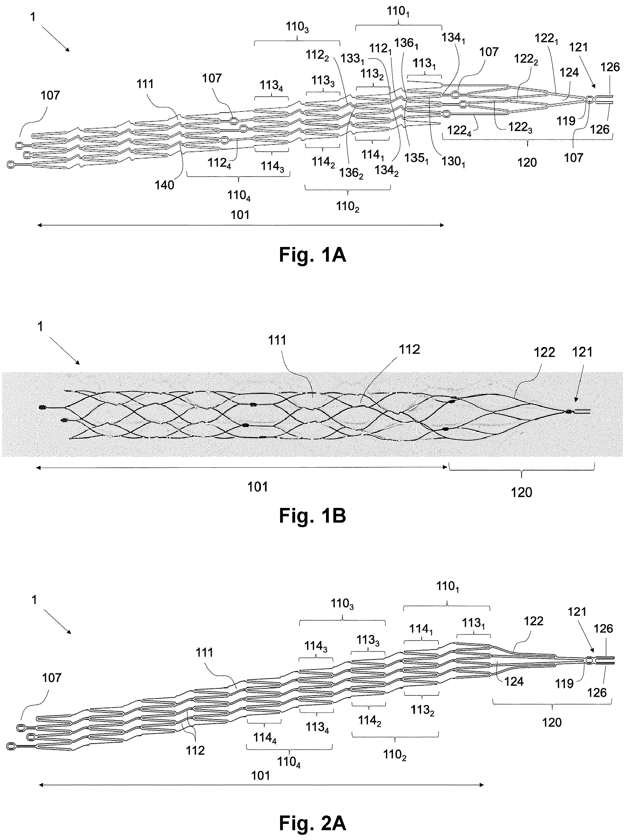

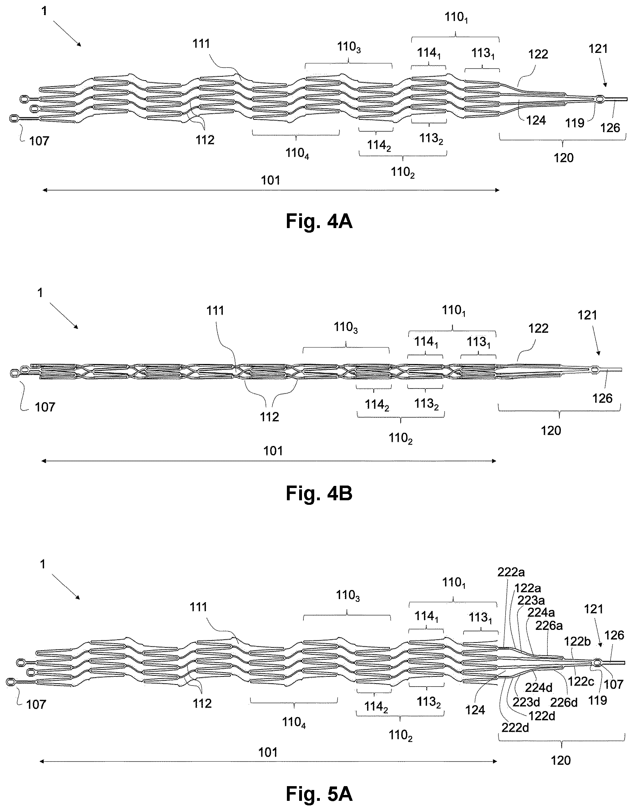

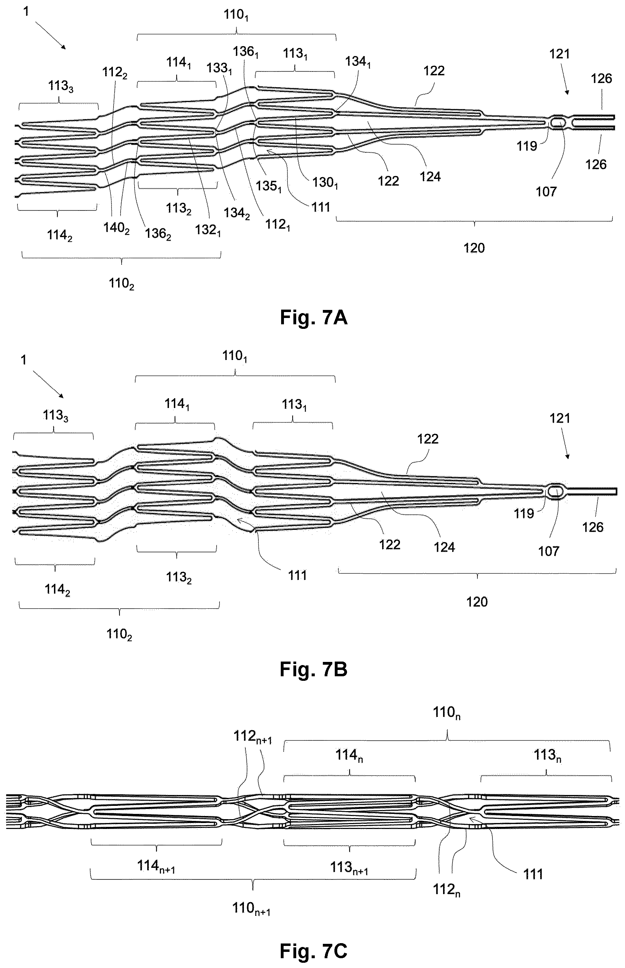

With reference to Figs. 1A-6B, therein different embodiments of the proposed clot mobilizer device 1 are illustrated. While the various embodiments all have longitudinally extending closed tubes with a tapered proximal end and an open distal end (as shown, e.g., in Fig. 4B), Figs. 1A, 2A, 3A, 4A, 5A, and 6A open and flatten particular embodiments to illustrate two-dimensionally the strut and cell patterns of those embodiments. It should be understood that the actual clot mobilizers corresponding to Figs. 1A-6B are longitudinally extending closed tubes defined by the illustrated struts and cells, in each case having a tapered proximal end and an open distal end, such as the embodiment shown in a compressed delivery form in Figs. 4B and 7C.

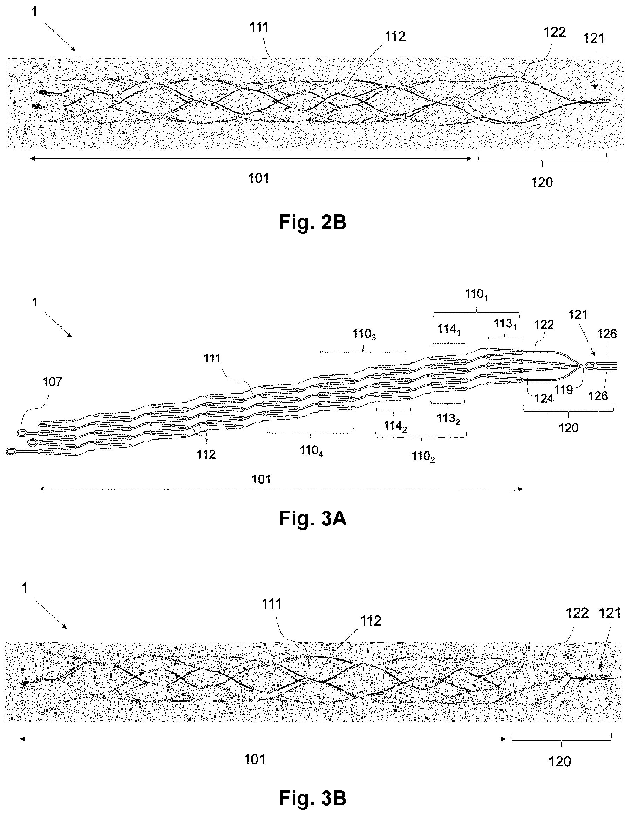

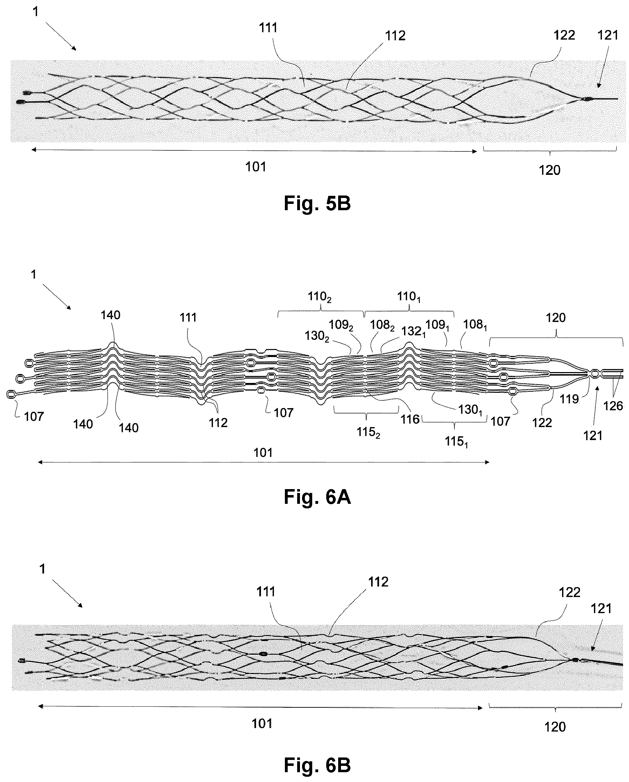

In at least some embodiments of the invention, the clot mobilizer device 1 includes a working portion 101 comprising a plurality of crowns 110₁ ...110ₙ of cells 111 defining a tubular-shaped section forming a cylindrically closed structure (as can be seen in the 3D representation illustrated in the embodiments of Figs. 1B, 2B, 3B, 4B, 5B, 6B, and 7C). For example, the number of crowns in the plurality of crowns 110₁ ...110ₙ can be between 4 and 10, more particularly 6, 7, 8 or 9 (with e.g., n = 1, 2, 3, 4, 5, 6, 7, 8, 9 or 10). As shown in Figs. 2A-B, 3A-B, 4A-B, and 5A-B, the working portion of the expandable clot mobilizer device comprises eight crowns of cells. However, the working portion can comprise seven crowns of cells (as shown in Figs. 1A-B) or nine crowns of cells (as shown in Figs. 6A-B). For purposes of this disclosure, a "crown" is a closed ring extending circumferentially around the device with alternating longitudinally longer and longitudinally shorter sections. Each crown of cells in the working portion can comprise, for example, four or six cells, and each crown can have components that are shared with one or more adjacent crowns. In some embodiments, the working portion 101 can have a length between 30 mm and 50 mm, for example approximately 40 mm.

Figs. 1A-B show an embodiment of the disclosure wherein each cell 111ₙ in the crowns of cells 110ₙ in the working portion 101 has an open area bordered by two proximal cell struts 130ₙ, two distal cell struts 132ₙ, and two middle cell struts 112ₙ. For purpose of the disclosure, "n" is used to reference interrelated features; for example, the first crown is referenced as n=1 and its respective adjacent crown as n+1=2, and successively. The distal cell struts 132₁ of the first crown 110₁ are disposed in a distal cell strut ring 114₁ at equal distal cell strut positions along a longitudinal axis of the clot mobilizer device 1, and the proximal cell struts 130₁ of the first crown 110₁ are disposed in a proximal cell strut ring 113₁ at equal positions along the longitudinal axis of the clot mobilizer device 1. In such crowns of cells, the proximal cell struts 130ₙ each extends distally from a proximal end 134ₙ common to one adjacent proximal cell strut to a distal end 136ₙ common to another adjacent proximal cell strut, where it joins a proximal end 135ₙ of a respective one of the two middle cell struts 112ₙ. Similarly, the distal cell struts 132ₙ each extends proximally from a distal end 136ₙ₊₁ common to one adjacent distal cell strut to a proximal end 134ₙ₊₁ common to another distal cell strut, where it joins a distal end 133ₙ of a respective one of the two middle cell struts 112ₙ. Each middle cell strut 112 in each crown borders two adjacent cells 111 in that crown. As shown in Figs. 1A-B, the distal strut ring 114ₙ of one crown of cells is the proximal strut ring 113ₙ₊₁ of the adjacent crown of cells, and the distal cell struts 132ₙ of distal strut ring 114ₙ are the proximal cell struts 130ₙ₊₁ of proximal strut ring 113ₙ₊₁.

The longitudinal and circumferential arrangements of the crowns of cells 110ₙ in the working portion 101 of the clot mobilizer device 1 can affect the behavior of the device in its unexpanded and expanded configurations. For example, providing a circumferential offset between adjacent crowns of cells can facilitate compression of the clot mobilizer from the expanded configuration into an unexpanded configuration for delivery through a catheter. This circumferential offset of adjacent crowns of cells can also reduce buckling and kinking during advancement of the clot mobilizer through the delivery catheter in its unexpanded delivery configuration. For example, in the working portion 101 of the embodiment shown in Figs. 1A-B, the common proximal ends 134ₙ₊₁ of each distal cell strut ring 114ₙ (i.e., the distal ends 133ₙ of middle cell struts 112ₙ) are circumferentially offset from the common distal ends 136ₙ of the proximal cell strut ring 113ₙ (i.e., the proximal ends 135ₙ of middle cell struts 112ₙ) in the same circumferential direction, such that the middle cell struts 112 between these common ends extend in a direction that is not parallel to the longitudinal axis of the working portion, as seen best in the detailed view of Fig. 7C; as explained before, for purpose of the disclosure, n=1 and n+1=2, and successively. Thus, the crowns are oriented one to each other in a helix pattern.

In the embodiment of Figs. 1A-B, each of the middle cell struts 112 is adapted and configured to be more flexible than the distal cell strut 132 and proximal cell strut 130 to which it extends. In this embodiment, the working portion 101 and tapered portion 120 of clot mobilizer device 1 are cut from a single tube and all elements have the same thickness (i.e., the tube thickness). As shown in the flattened view of Fig. 1A (which shows the pattern to be cut from the solid tube to make the clot mobilizer device 1), the middle cell struts 112 bordering each cell 111 have a width less than a width of the distal cell struts 132 bordering that cell 111 and less than a width of the proximal cell struts 130 bordering that cell 111, thereby making the middle cell struts more flexible than the proximal cell struts and the distal cell struts. For example, the distal cell struts 132 and proximal cell struts 130 can have substantially the same width, e.g., in the range between 0.10 mm and 0.145 mm, particularly 0.10 mm, 0.12 or 0.125 mm, while the middle cell struts can have a width in the range between 0.05 mm 0.13 mm, particularly 0.05, 0.08, 0.09 or 0.10.

To enhance the flexibility of the clot mobilizer device 1, each middle cell strut 112 in the working portion 101 also has hinge portions 140 to provide additional flexibility to the middle cell strut. As shown, the hinge portions 140 are preformed curves or bends in the middle cell strut 112 disposed between the proximal and distal ends of the middle cell strut 112. In this embodiment, the hinge portions 140 in some middle struts 112 are two bends formed closer to the proximal end of the middle cell strut than to the distal end, as shown in Fig. 1A. In other embodiments, each middle cell strut 112 in the working portion 101 can comprise fewer or more hinge portions 140. The hinge portion 140 can be integral with the middle cell strut, as shown in Figs. 1A-B, forming, e.g., a living hinge in which the hinge portion 140 and the other portions of the middle cell strut are parts of the same structure. For example, each hinge portion 140 can have an integral section of increased curvature and/or a bend. The bend forming the hinge provides added flexibility to the middle cell strut. The additional flexibility of the middle cell struts helps the clot mobilizer adapt to the shape of the vascular anatomy, e.g., as it is advanced and withdrawn in its unexpanded delivery configuration. The hinges also help the clot mobilizer device 1 to adapt its shape to the vascular anatomy into which it is expanded to its expanded configuration to capture a clot. In addition, this feature helps avoid kinking and buckling during advancement by providing multiple bending points along the length of the clot mobilizer device 1 to distribute the advancement force when the device encounters resistance to forward movement. Note that in some embodiments not all of the crowns of cells have hinge portions. For example, middle cell struts 112₄ in crown of cells 110₄ of clot mobilizer device 1 shown in Figs. 1A-B lack hinge portions.

The clot mobilizer device 1 can be made of Nitinol, cobalt-chromium alloys, iron alloys such as stainless steel or spring steel, etc.

The clot mobilizer device 1 illustrated in Figs. 1A-B also include radiopaque markers 107 at a distal end of the working portion 101, along middle cell struts 112₄ of the working portion 101, and in the tapered portion 120. In some embodiments, the different radiopaque markers 107 have different lengths to avoid entanglement between them or other devices. The radiopaque markers 107 can be made of a Platinum Iridium alloy or of Tantalum. The radiopaque markers 107 may be used under fluoroscopy to show the position and degree of expansion of clot mobilizer device 1.

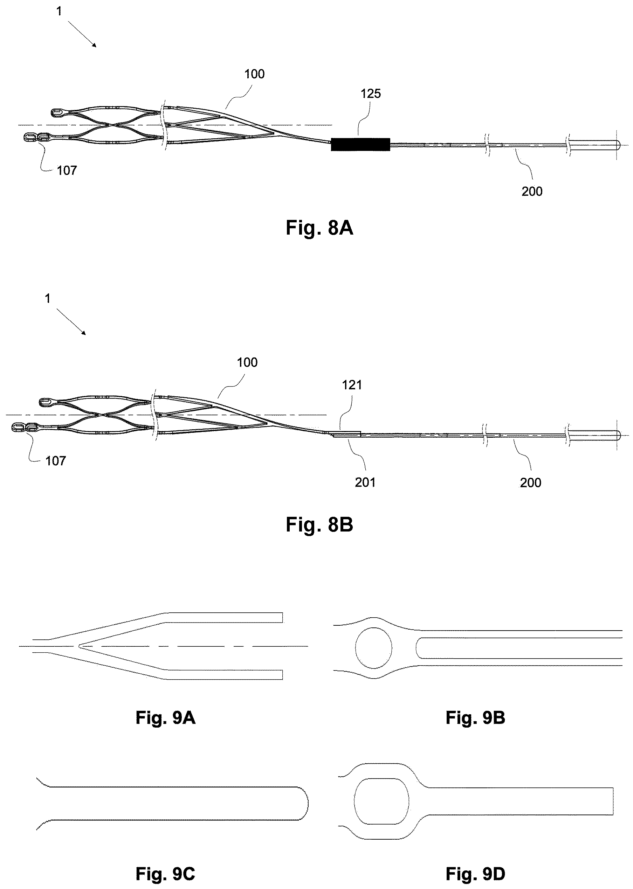

The clot mobilizer device 1 of Figs. 1A-B also includes a tapered portion 120 that extends proximally from a proximal end of the working portion 101 to a connection portion 121. The tapered portion 120 has a plurality of struts 122ₙ. At least some of the plurality of tapered portion struts 122 border a plurality of tapered portion cells 124. Particularly, the struts 122 of the tapered portion 120 can have a width greater than a width of the working portion struts, e.g., the distal cell struts 132, the proximal cell struts 130 and/or the middle cell struts 112 (e.g., in embodiments showed in Figs. 4A and 5A), thereby making the tapered portion struts stiffer (i.e., less flexible) than the struts of the working portion. The taper of the tapered portion 120 is achieved by the size and number of cells 124 in the tapered portion 120 compared to the sizes and numbers of cells in the working portion 101, with only a single tapered portion cell 124 where struts 122 converge to a connection point 119 distal to a radiopaque marker 107 at a distal end of a proximal connection portion 121. In the embodiment shown in Fig. 1A, four tapered portion struts 122₄ extend proximally from four common proximal ends 134₁ of the proximal struts 130₁ at the proximal end of working portion 101. Radiopaque markers 107 are disposed along three of these four struts 122₄. Two of the struts 122₄ extend to common distal ends of two pairs of tapered portion struts 122₃, while the other two struts 122₄ extend proximally to proximal ends of two of the four struts 122₃. The other two struts 122₃ extend to a common proximal end. Together tapered portion struts 122₃ and 122₄ define three tapered portion cells 124. Four tapered portion struts 122₂ extend proximally from proximal ends of struts 122₃ to two common proximal ends to define two tapered portion cells 124, and two tapered portion struts 122₁ extend proximally from the common proximal ends of struts 122₂ to a common proximal end just distal to the radiopaque marker 107. The tapered portion struts help at least the proximal end of the working portion provide a radially outward force due to, e.g., the tapered portion strut angle and/or thickness and the shapes of the cells they define. The tapered portion struts also help with pushability by effectively transmitting a distally-directed advancement force to the working portion of the clot mobilizer device 1. The proximal connection portion 121 can comprise two proximally extending arms 126. Arms 126 may connect to a pusher, as described with respect to Figs. 8-12 below.

Figs. 2A-B and 7A illustrate another embodiment of a clot mobilizer device 1 according to this disclosure.

As in the embodiment of Figs. 1A-B, the clot mobilizer 1 of Figs. 2A-B and 7A has a working portion 101 with crowns of cells 110ₙ defined by proximal cell strut rings 113ₙ distal cell strut rings 114ₙ, and middle cell struts 112ₙ extending between common distal ends 136ₙ (i.e., the proximal ends 135ₙ of middle cell struts 112ₙ) of proximal cell struts 130ₙ and common proximal ends 134ₙ₊₁ (i.e., the distal ends 133ₙ of middle cell struts 112ₙ) of distal cell struts 132ₙ. The distal cell struts of the first crown 110, are disposed in a distal cell strut ring 114₁ at equal distal cell strut positions along a longitudinal axis of the clot mobilizer device 1, and the proximal cell struts of the first crown 110₁ are disposed in a proximal cell strut ring 113₁ at equal positions along the longitudinal axis of the clot mobilizer device 1. For purpose of the disclosure, the first crown is referenced as n=1 and the next crown n+1=2, and successively.

Once again, the middle cell struts 112 are more flexible than the proximal cell struts 130 and the distal cell struts 132. As in the prior embodiment, the working portion 101 and tapered portion 120 of clot mobilizer device 1 are cut from a single tube, and all elements have the same thickness (i.e., the tube thickness). As shown in the flattened view of Fig. 2A (which shows the pattern to be cut from the solid tube to make the device), the middle cell struts 112 each have a width less than the width of the proximal cell struts 130 and the width of the distal cell struts 132, thereby making the middle cell struts more flexible than the proximal cell struts and the distal cell struts. For example, the distal cell struts 132 and proximal cell struts 130 can have substantially the same width, e.g., in the range between 0.10 mm and 0.145 mm, particularly 0.10 mm, 0.12 or 0.125 mm, while the middle cell struts can have a width in the range between 0.05 mm 0.13 mm, particularly 0.05, 0.08, 0.09 or 0.10. Unlike the embodiment of Figs. 1A-B, however, in the embodiment of Figs. 2A-B and 7A all middle cell struts 112 have hinge portions 140. In addition, the hinge portions 140 of this embodiment include two integral bends, one near the common distal end of the proximal cell struts from which the middle cell strut extends and other near the common proximal end of the distal cell struts to which the middle cell strut extends.

Like the embodiment of Figs. 1A-B, in the embodiment of Figs. 2A-B the common proximal ends 134ₙ₊₁ of each distal cell strut ring 114ₙ (i.e., the distal ends 133ₙ of middle cell struts 112ₙ) are circumferentially offset from the common distal ends 136ₙ of the proximal cell strut ring 113ₙ (i.e., the proximal ends 135ₙ of middle cell struts 112ₙ)in the same circumferential direction, such that the middle cell struts 112ₙ between these common ends extend in a direction that is not parallel to the longitudinal axis of the working portion. Thus, the crowns are oriented one to each other in a helix pattern.

The tapered portion 120 in the embodiment of Figs. 2A-B has four tapered portion struts 122, two of which extend proximally from the proximal end of the working portion 101 to meet at a point 119 distal to a radiopaque marker 107 and two proximally extending arms 126 of a connection portion 121(arms 126 may connect to a pusher, as described with respect to Figs. 8-12 below.). The other two struts 122 extend proximally from the proximal end of the working portion to intersect central portions of the first two struts 122. Together, the four struts define three tapered portion cells 124. Once again, the taper of the tapered portion 120 is achieved by the size and number of cells 124 in the tapered portion 120 compared to the sizes and numbers of cells in the working portion 101. The tapered portion struts help at least the proximal end of the working portion provide a radially outward force due to, e.g., the tapered portion strut angle and/or thickness and the shapes of the cells they define. The tapered portion struts also help with pushability by effectively transmitting a distally-directed advancement force to the working portion of the clot mobilizer device 1.

Three additional radiopaque markers 107 are disposed at the distal end of the working portion 101.

Figs 3A-B show yet another embodiment of a clot mobilizer 1 according to this disclosure.

Similar to the embodiment of Figs. 2A-B, in this embodiment has a working portion 101 with crowns of cells 110ₙ defined by proximal cell strut rings 113ₙ distal cell strut rings 114ₙ, and middle cell struts 112ₙ extending between common distal ends 136ₙ of proximal cell struts 130ₙ and common proximal ends 134ₙ₊₁ of distal cell struts 132ₙ. The distal cell struts of the first crown 110₁ are disposed in a distal cell strut ring 114₁ at equal distal cell strut positions along a longitudinal axis of the clot mobilizer device 1, and the proximal cell struts of the first crown 110₁ are disposed in a proximal cell strut ring 113₁ at equal positions along the longitudinal axis of the clot mobilizer device.

Once again, the working portion 101 and tapered portion 120 of clot mobilizer device 1 are cut from a single tube, and all elements have the same thickness (i.e., the tube thickness). As shown in the flattened view of Fig. 3A (which shows the pattern to be cut from the solid tube to make the device), the middle cell struts 112 each have a width less than the width of the proximal cell struts 130 and the width of the distal cell struts 132, thereby making the middle cell struts more flexible than the proximal cell struts and the distal cell struts. For example, the distal cell struts 132 and proximal cell struts 130 can have substantially the same width, e.g., in the range between 0.10 mm and 0.145 mm, particularly 0.10 mm, 0.12 or 0.125 mm, while the middle cell struts can have a width in the range between 0.05 mm 0.13 mm, particularly 0.05, 0.08, 0.09 or 0.10. The hinge portions 140 of this embodiment include two integral bends, one near the common distal end 136 of the proximal cell struts 130 from which the middle cell strut 112 extends and other near the common proximal end 134 of the distal cell struts 132 to which the middle cell strut 112 extends.

Like the embodiments of Figs. 1A-B and Figs. 2A-B, in the embodiment of Figs. 3A-B the common proximal ends 134ₙ₊₁ of each distal cell strut ring 114ₙ (i.e., the distal ends 133ₙ of middle cell struts 112ₙ) are circumferentially offset from the common distal ends 136ₙ of the proximal cell strut ring 113ₙ (i.e., the proximal ends 135ₙ of middle cell struts 112ₙ) in the same circumferential direction, such that the middle cell struts 112ₙ between these common ends extend in a direction that is not parallel to the longitudinal axis of the working portion. Thus, the crowns are oriented one to each other in a helix pattern.

The tapered portion 120 in the embodiment of Figs. 3A-B has five tapered portion struts 122, two of which extend proximally from the proximal end of the working portion 101 to meet at a point 119 distal to a radiopaque marker 107 and two proximally extending arms 126 of a connection portion 121 (arms 126 may connect to a pusher, as described with respect to Figs. 8-12 below.). Two other struts 122 extend proximally from the proximal end of the working portion to a common proximal end, and the fifth tapered portion strut 122 extends proximally from this common proximal end to point 119. Together, the five struts define three tapered portion cells 124. Once again, the taper of the tapered portion 120 is achieved by the size and number of cells 124 in the tapered portion 120 compared to the sizes and numbers of cells in the working portion 101. The tapered portion struts help at least the proximal end of the working portion provide a radially outward force due to, e.g., the tapered portion strut angle and/or thickness and the shapes of the cells they define. The tapered portion struts also help with pushability by effectively transmitting a distally-directed advancement force to the working portion of the clot mobilizer device 1.

Three additional radiopaque markers 107 are disposed at the distal end of the working portion 101.

Figs. 4A-B and 7B illustrate still another embodiment of a clot mobilizer device 1 of this disclosure. In this embodiment, a working portion 101 with crowns of cells 110ₙ defined by proximal cell strut rings 113ₙ distal cell strut rings 114ₙ, and middle cell struts 112ₙ extending between common distal ends 136ₙ of proximal cell struts 130ₙ and common proximal ends 134ₙ₊₁ of distal cell struts 132ₙ. The distal cell struts of the first crown 110₁ are disposed in a distal cell strut ring 114₁ at equal distal cell strut positions along a longitudinal axis of the clot mobilizer device 1, and the proximal cell struts of the first crown 110₁ are disposed in a proximal cell strut ring 113₁ at equal positions along the longitudinal axis of the clot mobilizer device 1.

Once again, the working portion 101 and tapered portion 120 of clot mobilizer device 1 are cut from a single tube, and all elements have the same thickness (i.e., the tube thickness). As shown in the flattened view of Fig. 4A (which shows the pattern to be cut from the solid tube to make the device), the middle cell struts 112 each have a width less than the width of the proximal cell struts 130 and the width of the distal cell struts 132, thereby making the middle cell struts more flexible than the proximal cell struts and the distal cell struts. For example, the distal cell struts 132 and proximal cell struts 130 can have substantially the same width, e.g., in the range between 0.10 mm and 0.145 mm, particularly 0.10 mm, 0.12 or 0.125 mm, while the middle cell struts can have a width in the range between 0.05 mm 0.13 mm, particularly 0.05, 0.08, 0.09 or 0.10. The hinge portions 140 of this embodiment include two integral bends, one near the common distal end of the proximal cell struts from which the middle cell strut extends and other near the common proximal end of the distal cell struts to which the middle cell strut extends.

In the embodiment of Figs. 4A-B and 7B the common proximal ends of the distal cell strut ring 114₁ of a first crown 110₁ of cells 111 of the plurality of crowns of cells 110₁ ...110ₙ (i.e., the proximal ends of the middle cell struts 112₁) are offset from the common distal ends of the proximal cell strut ring 113₁ of the first crown 110₁ of cells 111 (i.e., the distal ends of the middle cell struts 112₁) in a first circumferential direction, and the common proximal ends of the distal cell strut ring 114ₙ of a second crown 110₂ of cells 111 of the plurality of crowns of cells (i.e., the proximal ends of the middle cell struts 112₂) are offset from the common distal ends of the proximal cell strut ring 113₂ of the second crown 110₂ of cells 111 (i.e., the distal ends of the middle cell struts 112₂) in a second circumferential direction opposite to the first circumferential direction. This pattern repeats with subsequent crowns of cells, such that the common distal ends of proximal cell strut rings are alternately offset in the first circumferential direction and in the second circumferential direction in each successive crown of cells. Thus, the crowns are configured one to each other in a zig-zag pattern.

The tapered portion 120 in the embodiment of Figs. 4A-B (and in more detail in Fig. 7B) has four tapered portion struts 122, two of which extend proximally from the proximal end of the working portion 101 to meet at a point 119 distal to a radiopaque marker 107 and a proximally extending arm 126 of a connection portion 121 (arm 126 may connect to a pusher, as described with respect to Figs. 8-12 below). The other two struts 122 extend proximally from the proximal end of the working portion to intersect central portions of the first two struts 122. Together, the four struts define three tapered portion cells 124. Once again, the taper of the tapered portion 120 is achieved by the size and number of cells 124 in the tapered portion 120 compared to the sizes and numbers of cells in the working portion 101. The tapered portion struts help at least the proximal end of the working portion provide a radially outward force due to, e.g., the tapered portion strut angle and/or thickness and the shapes of the cells they define. The tapered portion struts also help with pushability by effectively transmitting a distally-directed advancement force to the working portion of the clot mobilizer device 1.

Three additional radiopaque markers 107 are disposed at the distal end of the working portion 101.

Figs. 5A-B illustrate an embodiment similar to the embodiment of Figs. 4A-B. In this embodiment, however, at least some of the taper portion struts 122 can have widths that vary over the length of the strut. Thus, for example, struts 122a and 122d have reduced width sections 222a and 222d at the distal end of the tapered portion 120, increased width sections 223a and 223d wider than, and proximal to, sections 222a and 222d, reduced width sections 224a and 224d narrower than, and proximal to, sections 223a and 223d, and increased width sections 226a and 226d wider than, and proximal to sections 224a and 224d and distal to their intersections with struts 122b and 122c, respectively. Struts 122b and 122c can also vary their widths along their lengths. Varying the width of at least some of the tapered portion struts 122 changes the way that the tapered portion 120 contributes to the radial outward force provided by the clot mobilizer 1. Other aspects of the embodiment of Figs. 5A-B are the same as in the embodiment of Figs. 4A-B.

Figs. 6A-B illustrate an embodiment of a clot mobilizer in which one or more crowns of cells lack flexible middle cell struts. For example, in this embodiment, a working portion 101 with crowns of cells 110ₙ defined by proximal cell strut rings 113ₙ, distal cell strut rings 114ₙ, and middle cell struts 112ₙ extending between common distal ends 136ₙ of proximal cell struts 130ₙ (i.e., the proximal ends 135ₙ of middle cell struts 112ₙ) and common proximal ends 134ₙ₊₁ of distal cell struts 132ₙ (i.e., the distal ends 133ₙ of middle cell struts 112ₙ) The distal cell struts 132ₙ of crown 110ₙ are disposed in a distal cell strut ring 114ₙ at equal distal cell strut positions along a longitudinal axis of the clot mobilizer device 1, and the proximal cell struts 130ₙ of crown 110ₙ are disposed in a proximal cell strut ring 113ₙ at equal positions along the longitudinal axis of the clot mobilizer device 1.

Once again, the working portion 101 and tapered portion 120 of clot mobilizer device 1 are cut from a single tube, and all elements have the same thickness (i.e., the tube thickness). As shown in the flattened view of Fig. 4A (which shows the pattern to be cut from the solid tube to make the device), the middle cell struts 112 each have a width less than the width of the proximal cell struts 130 and the width of the distal cell struts 132, thereby making the middle cell struts more flexible than the proximal cell struts and the distal cell struts. For example, the distal cell struts 132 and proximal cell struts 130 can have substantially the same width, e.g., in the range between 0.10 mm and 0.145 mm, particularly 0.10 mm, 0.12 or 0.125 mm, while the middle cell struts can have a width in the range between 0.05 mm 0.13 mm, particularly 0.05, 0.08, 0.09 or 0.10. The hinge portions 140 of this embodiment include three integral bends, one near the common distal end of the proximal cell struts from which the middle cell strut extends, another bend near the common proximal end of the distal cell struts to which the middle cell strut extends, and a third and much greater bend in the center of each middle cell strut.

The working portion 101 of the clot mobilizer device 1 illustrated in Fig. 6A-B can also include at least one crown 115 of intermediate cells 116, and each intermediate cell 116 can comprise an open area bordered by two proximal cell struts 108 intersecting with two distal cell struts 109, i.e., omitting the more flexible middle cell struts included in some of the other crowns of cells. The distal cell struts 109 of each intermediate cell 116 in the at least one crown 115 of intermediate cells 116 can comprise the proximal cell struts 130 of cells 111 in an adjacent crown 110 of cells 111, and the proximal cell struts 108 of each intermediate cell 116 in the at least one crown 115 of intermediate cells 116 can comprise the distal cell struts 132 of cells 111 in an adjacent crown of cells 110.

In the embodiment of Fig. 6A-B the common proximal ends of the proximal cell struts 108₁ in a first crown of intermediate cells 115₁ are offset from the common distal ends of the distal cell struts 109₁ in a first circumferential direction (i.e., a direction that is not parallel to the longitudinal axis of the working portion), and the common proximal ends of the proximal cell struts 108₂ in a second crown of intermediate cells 115₂ are offset from the common distal ends of the distal cell struts 109₂ in a second circumferential direction (i.e., a direction that is not parallel to the longitudinal axis of the working portion), opposite to the first circumferential direction. This pattern repeats with subsequent crowns of intermediate cells, such that the common proximal ends of proximal cell struts are alternately offset from common distal ends of the distal cell struts in the first circumferential direction and in the second circumferential direction in each successive crown of intermediate cells. Thus, the crowns are configured one to each other in a zig-zag pattern. Thus, the cells 110 disposed between successive crowns of intermediate cells are configured as inflexion points in terms of the opposite circumferential direction between successive crowns of intermediate cells.

The tapered portion 120 in the embodiment of Fig. 6A-B has nine tapered portion struts 122. Six of the tapered portion struts 122 extend proximally from the common proximal ends of the proximal struts 108 of the first crown of intermediate cells 115₁ to meet at three common proximal ends. Three radiopaque markers 107 are disposed in three of these six tapered portion struts 122. Three more tapered portion struts extend proximally from the three common proximal ends of these six tapered portion struts to meet at a point 119 distal to a radiopaque marker 107 and a pair of proximally extending arms 126 of a connection portion 121 (arms 126 may connect to a pusher, as described with respect to Figs. 8-12 below.). Together, the nine struts define five tapered portion cells 124. Once again, the taper of the tapered portion 120 is achieved by the size and number of cells 124 in the tapered portion 120 compared to the sizes and numbers of cells in the working portion 101. The tapered portion struts help at least the proximal end of the working portion provide a radially outward force due to, e.g., the tapered portion strut angle and/or thickness and the shapes of the cells they define. The tapered portion struts also help with pushability by effectively transmitting a distally-directed advancement force to the working portion of the clot mobilizer device 1.

Three radiopaque markers 107 are disposed at the distal end of the working portion 101, and three radiopaque markers 107 are disposed in middle cell struts 112₃.

In some embodiments, the working portion 101 has a compressed configuration with a first diameter of less than 1.5 mm and an expanded configuration with a second diameter of at least 3.0 mm. The working portion 101 can exert an outward radial force between 0.5 N and 3.0 N at every diameter between and including the first diameter and the second diameter. In a particular embodiment, the outward radial force exerted by the working portion 101 in a first diameter of 1.5 mm is between 1.4 N and 3.0 N, more particularly 1.4 N, 1.5 N, 2 N or 2.5 N; and the outward radial force exerted by the working portion 101 in a second diameter of 3.5 mm is between 0.5 N and 1.5 N, more particularly 0.6 N, 1 N, 1.4 N or 1.5 N.

In an embodiment, the working portion is configured to have a compressed configuration with a first diameter of less than 1.8 mm and to exert an outward radial force between 1.25 N and 2.5 N, particularly 1.25 N, 2.0 N. 2.2 N or 2.35 N when the first diameter is around 1.8 mm.

In some embodiments, the working portion is configured to exert an outward radial force between 0.5 N and 3 N in a diameter between 1.5 mm and 3.5 mm. In other embodiments, the working portion is configured to exert an outward radial force between 1.0 N and 2.0 N in a diameter between 2.0 mm and 3.0 mm. In a particular embodiment, the working portion is configured to exert an outward radial force between 1 N and 2 N in a diameter around 2.0 mm. In another embodiment, the working portion is configured to exert an outward radial force between 1 N and 2 N in a diameter around 3.0 mm.

In order to calculate the outward radial force, in an embodiment, e.g., a time-diameter variation can be used. To that end, the clot mobilizer device 1 is introduced in a 12 segments head (in particular a RX550 from Machine Solutions Inc. as further detailed in the below examples) that allows compressing the device uniformly, with very low friction force. The temperature is set to 37±2 ºC and the radial force bench decreases and increases the diameter according to specified profile, recording a radial force vs diameter curve.

**Table 1 indicates some of the features of the clot mobilizer device 1 illustrated in Figs. 1A-6B.**

| **Prototype ID** | **Radiopaque Marker Quantity and Location** | **Radiopaque Marker Form** | **Attachment Design** |
|---|---|---|---|
| ID15 - Fig. 1A | 3 distal + 3 middle + 1 proximal + 1 tapered | Oval shaped | U-shape |
| ID16 - Fig. 6A | | | |
| ID23 Fig. 2A | 3 distal + 1 tapered | Oval shaped | U-shape |
| ID24 Fig. 3A | | | |
| ID32 Fig. 4A | 3 distal + 1 tapered | Oval shaped | Needle |
| ID33 Fig. 5A | | | |

Table 1. Clot mobilizer device features. distal = distal section of the working portion; middle = middle section of the working portion; proximal = proximal section of the working portion; tapered = tapered portion.

Figs. 8A and 8B schematically illustrate a clot mobilizer device 1 according to some embodiments of the present disclosure.

According to these embodiments, the proposed clot mobilizer device 1 comprises an elongated device 100, which is formed by the cited working portion 101 and tapered portion 120 and is attached to a pusher 200, forming an attachment (assemblage) 125, see Fig. 8A. As shown in Fig. 8B, the proposed clot mobilizer device 1 comprises an elongated device 100 and a pusher 200, where the elongated device 100 is attached to the pusher 200 via the first (or proximal) connection portion 121 and a second connection portion 201.

Figs. 9A-9D illustrate different types of connection portions 121. The elongated device 100 of the clot mobilizer device 1 can comprise a first connection portion 121 extending proximally, the first connection portion 121 having a first attachment surface 102 (see Fig. 5). The pusher 200 can comprise a second connection portion 201 extending distally, the second connection portion 201 having a second attachment surface 202 facing and extending longitudinally aligned to the first attachment surface 102 to define an overlapping portion and to define first and second seams extending longitudinally along first and second lateral extents of the overlapping portion.

The clot mobilizer device 1 also comprises a first weld attaching the first and second connection portions 121, 201, extending from the first seam toward the second seam, and a second weld attaching the first and second connection portions 121, 201, extending from the second seam toward the first seam.

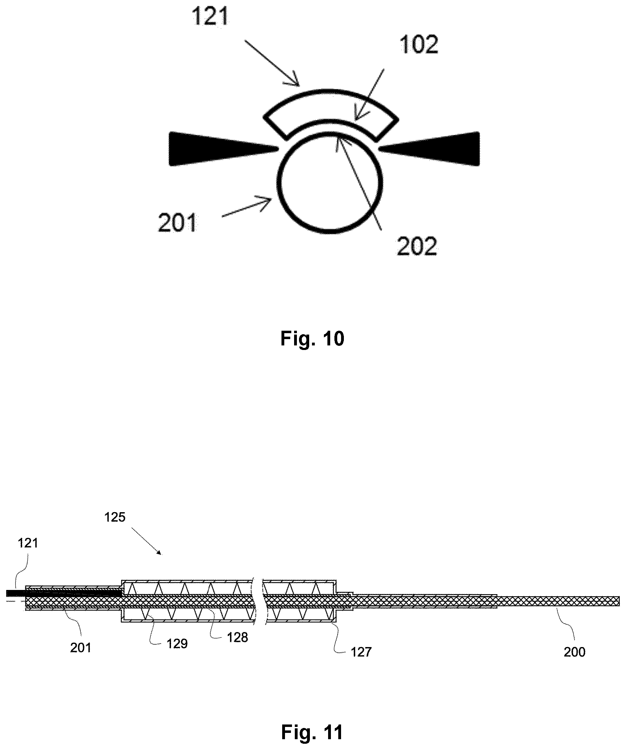

Fig. 10 illustrates an embodiment of the cross-sectional shape of the first connection portion 121 being a section of an annulus and of the cross-section shape of the second connection portion 201 being a circle. The black triangles show the direction of how the energy, particularly laser energy, is laterally applied to form the first and second welds. In this embodiment, the radius of the second attachment surface 202 is smaller than the radius of the first attachment surface 102.

Particularly, the ratio of the cross-sectional area of the first connection portion 121 perpendicular to the longitudinal axis to the corresponding cross-sectional area of the second connection portion 201 perpendicular to the longitudinal axis is in the range of 1:4 to 2:1 ; more particularly the ratio is 3:4.

The first weld can comprise multiple welding points along the first seam. Likewise, the second weld can also comprise multiple welding points along the second seam. In an embodiment, the welding points comprise a number between 2 and 12 welding points. In another embodiment, the welding points comprise a number between 2 and 5. The welding points are particularly aligned with each other. In some embodiments, the first weld and the second weld each can additionally comprise a welding seam disposed/arranged over the multiple welding points previously applied and formed by a plurality of consecutive welding points.

In some embodiments, the first weld and the second weld each extend along an entire length of the overlapping portion.

In some embodiments, glue can be added over the first and second welds.

Fig. 11 illustrates a particular embodiment of how the elongated device 100 connects to the pusher 200 by means of the attachment 125. According to this particular embodiment, the clot mobilizer 1 can comprise a jacket 127 or outer jacket, a jacket 128 or inner jacket, and a radiopaque coil 129 as a radiopaque element extending around the pusher 200, wherein the radiopaque coil 129 is arranged between the outer and inner jackets 127, 128 respectively. The jacket 128 extends proximally from the overlapping portion over a portion of the pusher 200. The jacket 127 extends around the radiopaque coil 129 and part of the overlapping portion. Additionally, the inner jacket 128 and outer jacket 127 can comprise a shrinkable material, for example a shrinkable plastic such as a thermoplastic.

Fig. 12 illustrates a method for associating (i.e., connecting/assembling) the elongated device 100 to the pusher 200 to enable the pusher 200 to maneuver the elongated device 100 (i.e., advance the elongated device 100 distally, withdraw the elongated device 100 proximally, and rotate the elongated device 100 about a longitudinal axis from a proximal end of the pusher 200). At step 401 the first attachment surface 102 of the first connection portion 121 is disposed longitudinally aligned to the second attachment surface 202 of the second connection portion 201 to form an overlapping portion and to form first and second seams extending parallel to the longitudinal axis along first and second lateral extents of the overlapping portion. At step 402 a first weld extending from the first seam towards the second seam is formed. At step 403 a second weld extending from the second seam toward the first seam is formed.

In some embodiments, step 402 comprises forming or applying a plurality of welding points (e.g., between 2 and 12, particularly between 2 and 5) extending from the first seam toward the second seam. Likewise, step 403 comprises forming or applying a plurality of welding points extending from the second seam toward the first seam. In some embodiments, the welding points of the plurality of welding points are aligned with each other. In some embodiments, steps 402 and 403 also comprise forming a welding seam by consecutively forming or applying a plurality of welding points on top of the previously formed/applied welding points.

To avoid accumulation of heat in specific zones, in some embodiments step 402 further comprises forming a first welding point of the plurality of welding points at a distal portion of the first seam and forming each other welding point of the plurality of welding points at a more proximal location along the first seam than a prior formed welding point of the plurality of welding points, and step 403 further comprises forming a first welding point of the plurality of welding points at a distal portion of the second seam and forming each other welding point of the plurality of welding points at a more proximal location along the second seam than a prior formed welding point of the plurality of welding points. That is, the energy device forming the weld is moved from the distal end of the first (and second) seam to the proximal end of said seam in order to avoid that the first connection portion 121, the second connection portion 102, or both, accumulate the heat which could provoke a breakage thereof.

In certain embodiments, the method includes covering a portion of the pusher 200 proximal to the overlapping portion with a jacket 128 (or inner jacket); placing a radiopaque element 129 at a proximal end of the overlapping portion and extending the radiopaque element 129 around the pusher 200; and covering the radiopaque element 129 with a jacket 127 (or outer jacket) and extending the later at least part of the overlapping portion.

In an embodiment, before inserting the inner jacket 128, the method can further comprise assembling, or placing, the radiopaque element 129 onto a distal end of the pusher 200 and pushing the radiopaque element 129 towards a proximal end of the pusher 200.

In another embodiment, before inserting the outer jacket 127, the method can further comprise placing, or pushing back, the radiopaque element 129 to the distal end of the pusher 200 (or at the proximal end of the overlapping portion), without covering the overlapping portion.

In some embodiments, before inserting the outer jacket 127, the method can further comprise heat shrinking the inner jacket 128 onto the overlapping portion, and after the outer jacket 127 has been inserted, heat shrinking the outer jacket 127 onto the inner jacket 128 and the radiopaque coil 129. The heat shrinking can be made using a suitable shrinkable material, e.g. a thermoplastic material such as polytetrafluoroethylene (PTFE); fluorinated ethylene propylene (FEP); perfluoroalkoxy alkanes (PFA); ethylene tetrafluoroethylene (ETFE); polyethylene terephthalate (PET) resins, etc.

In other embodiments, the elongated device 100 can be associated (i.e., connected/assembled) to the pusher 200 by other suitable attachment techniques, such as chemical or thermally bonding, friction or mechanically fitting, crimping, soldering, brazing, or even by using a connector material or member, or combinations thereof. In another embodiment, the elongated device 100 can be associated to the pusher 200 by crimping a coated or plated band disposed around and crimped to the overlapping portion.

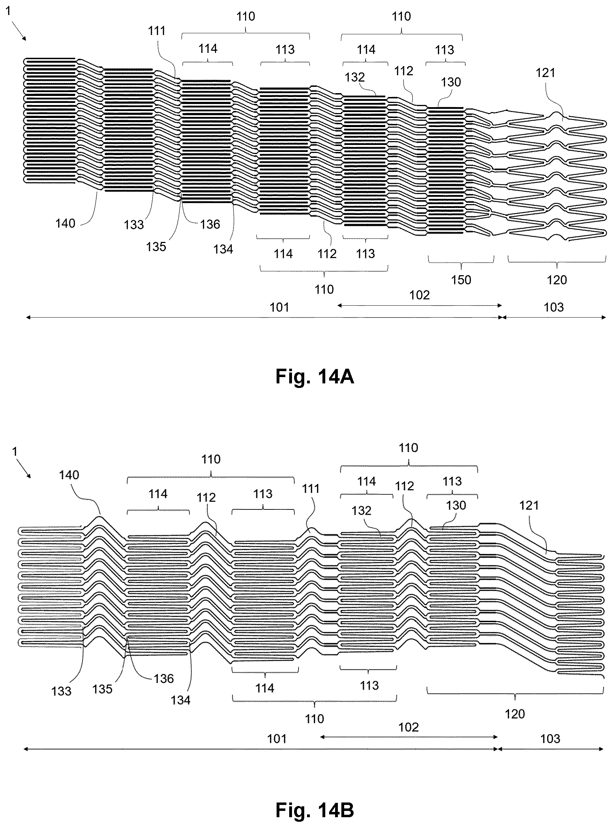

With reference to Figs. 14A and 14B, therein other embodiments of the proposed device are shown, in which the clot mobilizer device 1, or simply device 1, is a funnel. The figures show the pattern to be cut from the solid tube to make the device 1. Same as for the other explained embodiments, in this case, the device 1 also has a working portion 101 and a connection portion 103 that extends proximally from a proximal end of the working portion 101 and that can be connected to a pusher 200, 300, extending proximally from the connection portion.

The working portion 101 described therein is arranged and configured to provide an outward radial force at every diameter between and including a diameter of a compressed configuration of the working portion 101 and a diameter of an expanded configuration of the working portion 101. In some embodiments, the working portion 101 can expand from the compressed configuration to the expanded configuration and to exert an outward radial force at every diameter between and including a diameter of the compressed configuration and a diameter of the expanded configuration.

Moreover, in these embodiments, the working portion 101 comprises a plurality of crowns 110 of cells 111 that form a tubular-shaped section forming a cylindrically closed structure. The connection portion 103, particularly, also has a tubular-shaped section forming a cylindrically closed structure. More in particular, in the embodiment of Fig. 14A, the working portion 101 has five crowns 110 of cells 111 whereas in the embodiment of Fig. 14B the working portion 101 has four crowns 110 of cells 111. This is not limitative, since in other embodiments, in this case not illustrated, the working portion 101 simply needs to comprise (at least) three crowns of cells, each having (at least) four cells.

Each of the cells of the working portion 101 has an open area bordered by two proximal cell struts 130, two distal cell struts 132 and two middle cell struts 112. The proximal cell struts 130 each extend distally from a common proximal end 134 to a proximal end 135 of a respective one of the two middle cell struts 112, and the distal cell struts 132 each extend proximally from a common distal end 136 to a distal end 133 of a respective one of the two middle cell struts 112. Each middle cell strut 112 in each crown border two adjacent cells 111 in that crown, and is adapted and configured to be more flexible than the distal cell strut 132 and proximal cell strut 130 to which it extends.

In some embodiments, the distal cell struts 132 of each cell 111 in each crown of cells can comprise the proximal cell struts 130 of cells 111 in a (posterior/subsequent) adjacent crown of cells. Alternatively or complementary, in some embodiments, the proximal cell struts 130 of each cell 111 in each crown of cells can comprise the distal cell struts 132 of cells 111 in a (anterior/previous) adjacent crown of cells.

In some embodiments, the middle cell struts 112 bordering each cell 111 particularly have a width less than a width of the distal cell struts 132 bordering that cell 111 and less than a width of the proximal cell struts 130 bordering that cell 111. More in particular, the middle cell struts 112 each can have a width greater than or equal to 0.05 mm and less than or equal to 0.15 mm, for example 0.075 mm, the distal cell struts 132 each can have a width greater than or equal to 0.08 mm and less than or equal to 0.20 mm, for example 0.09 mm, and the proximal cell struts 130 each can have a width greater than or equal to 0.08 mm and less than or equal to 0.20 mm, for example 0.09 mm. The distal cell struts 132 and the proximal cell struts 130 can have substantially the same width.

As seen in the figures, the middle cell struts 112 integrate one or two hinge portions 140, having a section of increased curvature, that is/are arranged/disposed between the proximal and the distal ends of the middle cell strut 112. In Fig. 1B, each hinge portion 140 further involves a bend that can be either arranged in the central portion of the middle cell strut 112 or closer to one end of the middle cell strut 112 than to another end of the middle cell strut 112. In other embodiments, the hinge portion 140 comprises two bends.

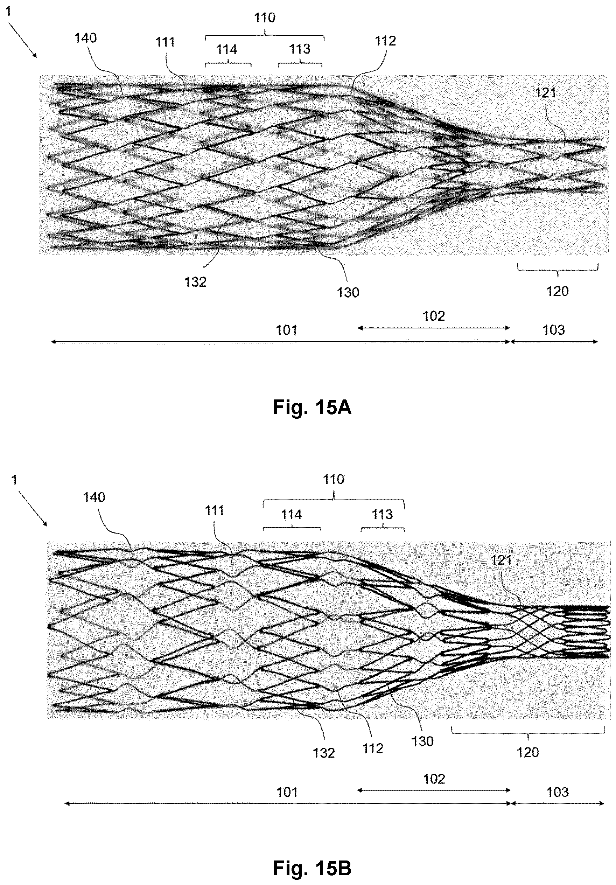

In each crown 110 of cells 111 of the working portion 101, the distal cell struts 132 are disposed in a distal cell strut ring 114 at equal distal cell strut positions along a longitudinal axis of the device 1; the proximal cell struts 130 are disposed in a proximal cell strut ring 113 at equal proximal cell strut positions along the longitudinal axis of the device 1; and the common distal ends of the distal cell strut ring 114 are circumferentially offset from the common proximal ends of the proximal cell strut ring 113, particularly, in one (see Fig. 14A or 15A) or more than one (see Figs. 14B or 15B) circumferential directions.

In some embodiments, the working portion 101 can also include at least one crown of intermediate cells, each intermediate cell comprising an open area bordered by two proximal cell struts intersecting with two distal cell struts. The distal cell struts of each cell in the at least one crown of intermediate cells can comprise the proximal cell struts of cells in an adjacent crown of cells. Similarly, the proximal cell struts of each cell in the at least one crown of intermediate cells can comprise the distal cell struts of cells in an adjacent crown of cells.

As can be seen in Figs. 15A and 15B, particularly, the working portion 101 also includes a tapered portion 102 at the proximal end thereof. The tapered portion 102 comprises a plurality of tapered portion struts and has an expanded diameter at a proximal end that is smaller than an expanded diameter of the working portion 101. In these embodiments, the tapered portion 102 has an expanded configuration that defines a frustoconical shape with a closed structure. Moreover, the tapered portion 102 includes one or more crowns of cells of the plurality of crowns 110 of cells 111. The tapered portion 102 converges at a distal end of the connection portion 103.

In the embodiment of Fig. 14A, the tapered portion 102 also includes a crown 150 of cells with each cell comprising an open area bordered by two proximal cell struts and two distal cells struts. In each cell, the proximal cell struts each extend distally from a common proximal end to proximal ends of the distal cell struts, the distal cell struts each extending distally from their proximal ends to a common distal end.

With regard to the connection portion 103, the latter comprises at least one crown 120 of cells 121, where each cell 121 has an open area bordered by two proximal cell struts, two distal cell struts, and two middle cell struts. The proximal cell struts each extend distally from a common proximal end to a proximal end of a respective one of the two middle cell struts. The distal cell struts each extend proximally from a common distal end to a distal end of a respective one of the two middle cell struts. Each middle cell strut border two radially adjacent cells, and is adapted and configured to be more flexible than the distal cell strut and proximal cell strut to which it extends. The connection portion 103 has an attachment surface 104 (or first attachment surface) to be connected to a pusher 200, 300.

The device 1 can be made of a metal, a metal alloy or a composite including Nitinol or Nitinol/Platinum. However, other types of metals or even other types of materials can be also used, for example cobalt-chromium alloys or iron alloys such as stainless steel or spring steel; also, other materials with shape memory properties can be used, for example cooper or magnesium alloys.

In some embodiments, the device 1 can be coated, for example, with a polymer material. In some embodiments, the coating is a hydrophilic or a hydrophobic material. In some embodiments, the coating can be a non-permeable coating. Alternatively, or complementarily, the coating can comprise an elastomeric or thermoplastic elastomer material such as polyurethane or silicone, among others. The coating can be disposed over the entire surface of the device 1, in other embodiments, the coating can just cover at least one of the working portion 101, the tapered portion 102 or the connection portion 103.

Following, different examples of the use and performance of the proposed clot mobilizer device 1 are detailed. The examples and drawings are provided herein for illustrative purposes, and without intending to be limiting to the present invention. The scope of the invention is defined by the claims.

### Example 1: In vitro 3D simulation model, Pre-Clinical Data

### Introduction

Endovascular treatment (EVT) is recognized as the most effective treatment for large vessel occlusion (LVO) strokes. Highest degree of recanalization in the shortest time with the minimum number of attempts has been demonstrated to correlate with improved clinical outcomes. Although highly effective, failure to reach complete recanalization has been reported in about 20% of treated patients. In order to improve patient outcomes, different devices and combinations are under development to increase the first pass complete recanalization rate. The development of such devices includes preclinical testing in phantom models simulating the cerebrovascular human anatomy, and animal models in which device related vessel injury can be assessed. Each simulation model has its own characteristics and therefore it is recommended that any new device or combination will prove its efficacy and safety in different conditions before final evaluation in a first in human study.

The clot mobilizer device 1 (hereinafter Conda) is a stent-like device, with the capacity of recanalize an occlusion from a blood vessel. In these embodiments, particularly, Conda is formed mainly by a stent and a pusher wire. The prototype used in this first example is the ID32/ID33 (Figs. 4-5) that presents a closed cylindrically working portion and a tapered portion that joints the stent with the pusher wire through a needle-shape attachment.

This study was aimed to assess the efficacy and performance of the Conda prototype in comparison with Trevo^{™} XP (4x30 mm, Stryker, hereinafter Trevo) and Solitaire^{™} 2 (4x40 mm, Medtronic, hereinafter Solitaire), the currently most used commercial stent retriever, in an *in vitro* 3D simulation model, a cerebrovascular model of the intracranial circulation that simulates the carotid and cerebral physiological blood flow, pressure and vessel anatomy including an occlusive *ex vivo* clot analog. Solitaire and Trevo are designed for use in the flow restoration of patients with ischemic stroke due to large intracranial vessel occlusion. They are stent-like devices, which can be fully deployed, fully resheated, and recovered. Solitaire and Trevo are available in variable diameter sizes: 4 and 6 mm, and 3, 4 and 6 mm; respectively.

In order to mimic the clinical scenario, the Clot Mobilizers (CMs) Conda, Solitaire and Trevo were used in this study in combination with the ANA Aspiration Catheter device (from Anaconda Biomed, hereinafter ANA), which is comprised of two coaxial catheters: the delivery catheter (comprises a hydrophilic coating to reduce friction and radiopaque markers on the distal end) and the aspiration catheter (comprised by a catheter with a funnel shape in the distal end, able to provide local flow restriction). However, the objective of the study is to compare the performance and efficacy of Conda, Solitaire and Trevo.

The main objectives of the study were:
- Assessment of the efficacy of Conda, Solitaire and Trevo in combination with ANA in terms of the rate of revascularization and rate of clot embolization.
- Assessment of the performance of Conda, Solitaire and Trevo in combination with ANA to reach the target vessel, to deploy the stent, and to withdrawal.

### Methods

Mechanical thrombectomies with ANA, in combination with the CMs (Conda, Solitaire and Trevo), were simulated in the model cerebrovascular occlusion (including a clot analog). The procedures were followed by a low-resolution fluoroscopy and assisted by trained technicians.

The cerebrovascular model system was composed of a human vascular replica and a physiologically relevant mock circulation flow loop. A three-dimensional *in vitro* model of the intracranial circulation was used as vascular replica.

The Vascular model Jacobs Institute V3.4 (JI V3.4) was used to simulate the mechanical thrombectomy intervention. This model was designed based on patient vascular anatomy using CT-A imaging (50 patients) and then printed on an 3D printer (Jacobs Institute). The model closely resembles the human intracranial circulation in terms of curvature, diameter, and length, and consists of the internal carotid artery segment and middle cerebral artery branches (M1-M4 segments), bilateral A1 anterior cerebral artery segments connected to a single anterior cerebral artery, and a single posterior communicating artery, thus allowing near complete circle of Willis circulation (intracranial model).

For the assessment of the efficacy in clot retrieval (revascularization and embolization rates), soft red and fibrin rich clots were used to generate middle cerebral artery (MCA; M1 & M2) occlusions.
- Soft red clot (5 x 8 mm): 4 ml of non-anticoagulated porcine blood was mixed with 32 mg of fibrinogen from bovine plasma (F8630, Sigma-Aldrich) and 1 unit of thrombin form bovine plasma (T4648, Sigma-Aldrich) for at least 3 min. The mixture was incubated at room temperature for at least 60 min.
- Fibrin rich clot (8 x 8 x 8 mm): Porcine blood was anticoagulated using sodium citrate solution (3.2%) immediately after collection. The whole blood constituents were subsequently separated using centrifugation (600g, 15 min, 4°C) and the extracted plasma was mixed with the red blood cells (RBCs) in a ratio of 9:1. Coagulation was initiated by the addition of calcium chloride (2.06%) and the clotted material was allowed to mature for 60 min at 37ºC. The resultant clots consist of approximately 100% fibrin.

The clots were injected into the flow loop to form an MCA occlusion. Prior to initiating thrombectomy, complete occlusion with TICI 0 was required. TICI scale is the standard score to assess the revascularization rate and is defined as follows:
- TICI scoring grade 0: no perfusion.
- TICI scoring grade 1: antegrade reperfusion past the initial occlusion but limited distal branch filling with little or slow distal reperfusion.
- TICI scoring grade 2- grade 2a: antegrade reperfusion of less than half of the occluded target artery previously ischemic territory (e.g., in one major division of the middle cerebral artery (MCA) and its territory).
- TICI scoring grade 2b: antegrade reperfusion of more than half of the previously occluded target artery ischemic territory (e.g., in two major divisions of the MCA and their territories).
- TICI scoring grade 3: complete antegrade reperfusion of the previously occluded target artery ischemic territory, with absence of visualized occlusion in all distal branches.

Other used guidance/support devices in combination with ANA, Conda, Solitaire and Trevo are shown in Table 2:

**Table 2. Support devices used in combination with ANA, Conda, Solitaire and Trevo during interventions.**

| **Device type** | | **Name** | **Company** |
|---|---|---|---|
| Neurovascular guide catheters | Guide catheter | Neuronmax 088 | Penumbra |
| | Microcatheter | Rebar 18 Micro Catheter | Covidien |
| | | Marksman Catheter | Medtronic |

### Procedure of mechanical thrombectomy

Neuron Max 088 guide catheter was placed in the cervical Internal Carotid Artery (ICA) and delivered the guidewire which was then softly advanced through the target vessel.

The ANA device was combined with the CMs independently to retrieve the clot: with the stented funnel deployed proximal to the occlusion, the microcatheter with the CM in it was navigated through the aspiration catheter and the deployed stented funnel over the wire until reaching and crossing the clot. The CM was deployed to capture the clot while continuous aspiration was applied via ANA (aspiration was applied after the microcatheter was retrieved), the CM was dragged until the whole clot was safety placed inside the stented funnel and both devices were finally withdrawn as one.

The revascularization rates were evaluated to assess the efficacy following the procedure time points after all procedure execution: pre-clot placement (for baseline), pre-treatment (clinical starting point) and post-thrombectomy (first, second and third passes of revascularization). TICI 2b and 3 are considered successful revascularization (1). TICI 0, 1, and 2a are considered unsuccessful revascularization (0). The main endpoints considered in the efficacy assessment were: Rates of revascularization after first and third passes (TICI 2b-3). Also, Distal Territory (EDT) and Emboli New Territory (ENT) were assessed. EDT score of 0 and ENT score of 0 is indicative of no embolic events. EDT score of 1 and ENT score of 1 is indicative of an embolic event. The procedure time points after all procedure execution were followed, and the main endpoints considered in the efficacy assessment were EDT and ENT after first and third passes (TICI 2b-3).

Sixteen interventions were carried out using sixteen different samples of Conda: eight samples of Conda (five of the ID32 and three of the ID33) were used for each type of clot: soft red clots and fibrin rich clots; ten samples of Solitaire were tested in soft red clots and fifteen in fibrin rich clots; finally, eleven samples of Trevo were tested in soft red clots and fifteen samples in fibrin rich clots.

The performance of Conda in the model JI V3.4, was assessed after the first attempt by studying the following endpoints depending on their quantification (i.e., score):
- IS-COMP: Conda Introducer Sheath (IS) compatibility with the Hemostatic Valve and Luer of the funnel; [1 = poor behavior, CM could be damaged during introduction; 2 = usual behavior compared to other brands; 3 = easy to introduce into the HYV and through the Luer but gets into the CM; 4 = easy to introduce into the HYV and through the Luer, does not get into the CM].
- C-PUSH: Pushability of the Conda to the target vessel. Access from the IS to the microcatheter; [1 = poor; 2 = moderate-adequate; 3 = very good].
- S-TARGET: Conda reaches the target zone; [1 = poor; 2 = moderate-adequate; 3 = very good].
- S-ACCURACY: Accuracy of the deployment of the Conda in the target vessel; [1 = poor accuracy, target vessel not reached; 2 = moderate accuracy target vessel not reached; 3 = moderate accuracy target vessel reached; 4 = very accurate, deployed in the target vessel].
- S-ADAPTABILITY: Conda adaptability (with the clot and target vessel); [1 = stent does not expand; 2 = stent self-expands but not completely; 3 = stent self-expands integrating the clot but not completely adapts to the vessel; 4 = stent self-expands integrating the clot and adapting to the vessel].
- C-VISIBILITY: Visibility of the Conda under fluoroscopy; [1 = poor; 2 = moderate-adequate; 3 = very good].
- S-F-ENTANGLEMENT: Compatibility of the funnel and the Conda; [1 = entanglement; 2 = few interactions; 3 = no interaction].
- S-RETRIEVAL: Resistance of the Conda during retrieval; [1 = friction during withdrawal; 2 = easy withdrawal].

Finally, a visual inspection to quantify the integrity of the Conda and the funnel of the aspiration catheter was performed after all procedure executions.

### Results and conclusions

The following Table 3 presents the results of the efficacy (revascularization rate) obtained comparing the use of Conda Solitaire and Trevo when ANA device is used:

**Table 3. Results of efficacy (revascularization rate) of Conda, Solitaire and Trevo in conjunction with ANA.**

| **CEREBROVASCULAR MODEL** | | **CLOT TYPE** | **ANA + Conda** | | | **ANA + Solitaire** | | | **ANA + Trevo** | | |
|---|---|---|---|---|---|---|---|---|---|---|---|
| **Model** | **Tortuosity Index** | | **n** | **1^{st} PASS** | **3^{rd} PASS** | **n** | **1^{st} PASS** | **3^{rd} PASS** | **n** | **1^{st} PASS** | **3^{rd} PASS** |
| JACOB S V3.4 | access: 5.464 | SOFT RED | 8 | 88% | 100% | 10 | 90% | 90% | 11 | 91% | 91% |
| | intracerebral: 3.859 | FIBRIN RICH | 8 | 88% | 100% | 15 | 67% | 73% | 15 | 87% | 100% |
| | global: 9.323 | | | | | | | | | | |

The results obtained with the Conda device in the first pass were similar to Solitaire and Trevo testing with soft red clot and superior to Trevo and Solitaire testing with fibrin rich clot, indicating a better efficacy of the Conda device in thrombectomy procedures; also, the results demonstrated a better efficacy after three passes using Conda. The highest differences were observed in the extraction of fibrin rich simulated thrombi. This type of thrombi is harder, and it has a higher tendency to be rolled-out from the stent during retrieval.

The efficacy (revascularization rate) obtained with the Conda device was higher than with the Solitaire when used with ANA device and similar to Trevo. These results imply that the Conda is an effective device for the extraction of clots, and it is the better choice than Solitaire when used together with the ANA device. In the following Table 4 the results of performance obtained with the different interventions using Conda are presented, e.g., the endpoint of pushability (C-PUSH) obtained a score of 3 in 16 interventions (100% of all interventions).

**Table 4. Performance endpoints scores of Conda after the first pass using the JI V3.4 model.**

| **Endpoint** | **% of interventions per score (number of interventions)** | | | | **Mean/Max. Score** |
|---|---|---|---|---|---|
| | **1** | **2** | **3** | **4** | |
| **IS-COMP** | 0% | 0% | 0% | 100% (16) | 4/4 |
| **C-PUSH** | 0% | 0% | 100% (16) | - | 3/3 |
| **S-TARGET** | 0% | 0% | 100% (16) | - | 3/3 |
| **S-ACCURACY** | 0% | 0% | 0% | 100% (16) | 4/4 |
| **S-ADAPATABILITY** | 0% | 0% | 0% | 100% (16) | 4/4 |
| **C-VISIBILITY** | 0% | 100% (16) | 0% | - | 2/3 |
| **S-F-ENTANGLEMENT** | 0% | 0% | 100% (16) | - | 2/2 |
| **S-RETRIEVAL** | 0% | 100% (16) | - | - | 1/1 |

All the end points evaluated showed that the Conda device has a very good behavior in reaching the target vessel, being adapted to the vessel wall, and without problems during the retrieval. The only endpoint in which was not possible to obtain the best qualification was in the visibility of the device under fluoroscopy. However, the visibility level obtained was considered acceptable but sub-optimal.

Finally, the compatibility of Conda device with ANA was studied by quantifying the integrity of the CM. As a result, no damage was found in the different Conda devices due to their use in conjunction with ANA device. It is considered that Conda device can be used with ANA without risk.

This study showed that Conda in conjunction with ANA obtained better results than Trevo and Solitaire in conjunction with ANA. These results imply that the Conda is an effective device for the extraction of clots, and it is the better choice than Solitaire or Trevo when used together with the ANA device. Conda also showed very good behavior in reaching the target vessel, being adapted to the vessel wall, and without problems during the retrieval. Finally, no damage was found in the Conda device due to their use in conjunction with ANA device, confirming that is totally compatible with ANA.

As conclusion, Conda is a clear option to be used in clinical trials to increase the revascularization rate during mechanical thrombectomies.

### Example 2: In vivo study of the Conda clot mobilizer prototypes: assessment of the safety and performance in the porcine model of thrombectomy

### Introduction

Different prototypes of the Conda were analyzed in this study. In these embodiments, particularly, Conda is formed mainly by a stent and a pusher wire.

This study aimed to assess the safety and performance of the Conda prototypes in the *in vivo* model of vessel injury and recanalization in porcine arteries, in order to test the vascular damage produced by the device to the vascular tissue and its performance reaching a target site on blood vessels of an animal swine model, and compare the clot mobilizer device with a similar marketed stent retriever (Solitaire^{™} 2 revascularization device, manufactured by Medtronic, hereinafter as Solitaire), as in Example 1.

This experimental study also aimed the evaluation of some aspects of the clot mobilizer performance and usability, including the device maneuverability in clot retrieval, although, this animal model is limited to assess navigability through highly tortuous complex vascular anatomy (such as in the human cerebral vasculature).

In order to mimic the clinical scenario, the Clot Mobilizers (CMs) Conda and Solitaire were used in this study in combination with a microcatheter and with ANA (already described in Example 1) or a standard guide catheter, to reach the target vessel.

This study was aimed to assess the following:
- The safety: any vascular injury at the renal arteries, triggered by the Conda prototypes in comparison with the marketed device Solitaire, both in combination with ANA or standard guide catheter, both systems after 3 passes.
- The performance (effectiveness) and usability of Conda prototypes, in comparison with Solitaire, in combination with ANA or standard guide catheter, when used under a simulated procedure and environment, with or without a clot occlusion in the vessel lumen.
- The radial force mechanical value that characterizes the force that the complete length of the stent will exert against the wall of the blood vessel.

### Methods

This study was performed in the *Animal Facility* of the *Institut de Recerca de Vall d'Hebron* (*VHIR*), Barcelona (Spain).

16 female swines with a weight between 30 and 35 Kg of the species *Sus scrofa* were used as experimental subjects. All of them without diseases and under healthy conditions and facilitated by A.M. Animalia Bianya S.L. (from Girona, Spain). Intervened animals were numbered as: C21, C22, C23, C25, C27, C29 and C31.

The renal arteries of the swines were treated with different devices and the distal branch of each renal artery that was not treated was used as negative control. In each animal, after renal artery interventions had been completed, a synthetized radiopaque thrombus was inserted in the subclavian artery or a branch of the carotid artery to generate an intentional vascular blockade, followed by a recanalization attempt to retrieve the clot with the CMs.

The porcine model is a model already established for the evaluation of neurovascular and intravascular devices and techniques. This model is also useful to assess mechanical thrombectomy systems indicated for patients with large ischemic stroke. This model mainly allows assessing the vascular damage exerted by the device during use (security), and it also provides information on the performance and usability of the device in an *in vivo* model (efficacy) including the thrombectomy approach, in arteries of similar diameter to the cerebral arteries, which provide information further on the adequacy of its design. These models are very good for assessing the usability of the device: navigability, maneuverability and ability to access the target vessel, the efficacy in the thrombus capture and retrieval, the assessment of thrombus fragmentation and embolization.

The clot mobilizers used in this study were designed for use in the neurovasculature, such as the internal carotid artery, M1 and M2 segments of the MCA, anterior cerebral artery, and the basilar and vertebral arteries. Clot mobilizers used in this study are defined in Table 1 and/or Figs. 1A-6B.

### Procedure

The intervention was performed in a surgical room equipped with a fluoroscopy, echography, anesthesia and monitoring equipment. The general management of the intervention was performed by the facility veterinarian staff, and the specific procedure with the neurothrombectomy devices was assisted by a specialized interventionalist. The intervention with the thrombectomy devices was followed by fluoroscopy.

Each animal procedure started with a vascular injury performance assessment, that consisted in the intervention to the cranial or caudal branch of the right/left renal arteries and performance of a device deployment without clot removal in the chosen branch, while the adjacent branch (cranial or caudal) of the same side in the renal circulatory system artery (not subjected to any intervention) was used as a negative control artery.

Each intervention (after ensuring access of the device to the target section of the artery) consisted of three repetitions deployment of the tested devices (CM) and the resheathing procedure on the target section of the artery (3 passes), simulating the maneuvers for the thrombus capture and retrieval (in absence of clot), and the withdrawal of the device, according to the instructions for use of the devices and the standard procedure provided in the clinical use.

In interventions with ANA combined with the CM, the funnel was expanded proximally, in the proximal segment of the renal artery (large vessel with 3-4.5 mm in diameter), allowing sufficient vessel length to deploy the clot mobilizer distally in the distal branch (small vessel with 2-3 mm in diameter). This allowed the evaluation of the compatibility of the funnel with the CM and the vessel injury caused by both devices. A similar procedure was followed when the tested devices were combined with only a standard guide catheter, but in this case the guide catheter distal end reached the proximal segment of the renal artery, and the CM was deployed distally in the distal branch and retrieved until reaching the proximal section of the renal artery. The general performance of devices including the devices preparative and compatibility, visibility, navigability, flexibility and pushability, withdrawal and devices integrity assessment were semi-qualitatively evaluated.

A fragment of an *ex vivo* radiopaque autologous clot was introduced into the left or right subclavian artery or a branch of the carotid artery (i.e., cervical artery) followed by a test device introduction and thrombectomy maneuver to attempt to retrieve the clot, according to directions for use.

To document the target vessel and the performance of the devices, several angiographies were registered during the intervention. Vascular response, including vasospasm, dissection, perforation or thrombosis, and revascularization was evaluated using angiographies. Angiographic images of the vessel were obtained with contrast media (Lopamidol, 10 ml/kg, 370 mg/ml) to identify the proper location for the deployment site. Additional angiograms may have been recorded at this point, or later on, during the procedure, at the discretion of the interventionalist. Baseline angiogram was performed to document the reference diameter for the device access and deployment. It was verified that the guide catheter was positioned in the target vessel. Baseline angiogram was performed to document the reference diameter for the device access and deployment. Post-procedural angiographies were registered to assess the vessel status and/or revascularization after clot retrieval (mTICI scale, defined in Example 1). Also, the general performance including the compatibility of the ANA with Conda were assessed.

The interventional procedure was carried out as follows:
- Vascular Access: After induction of anesthesia, the right femoral artery was accessed through an incision made in the inguinal region. An arterial short 8F sheath was introduced and advanced into the artery, under echography guidance. A venous blood sample was collected prior to heparin administration and fluid therapy initiation.
- Vessel Angiography: Under fluoroscopic guidance, a 6F guide catheter-long sheath (Neuron Max) was inserted through the sheath using a 5F diagnostic catheter Simmons type I and a wire guide, and advanced to the target location: the cranial branch of the left renal artery. Guide catheter was continuously flushed with saline solution. Then, the diagnostic catheter and guide wire was removed.
- Clot embolization: A carotid artery branch or subclavian artery (section with a diameter approx. 3-4 mm) was embolized with an *ex vivo* autologous radiopaque clot of 4-4.5 mm diameter 5-20 mm length (synthetized by personnel in the animal facility VHIR according to internal patented procedure) contained in a 1 ml syringe. A 6F guide catheter was located in the target artery, then the syringe containing the clot was connected and the clot injected distally in the target artery. The guide catheter was removed for 5-10 minutes to reestablish the flow and allow the clot stabilization within the artery lumen. Hereinafter the guide catheter was reintroduced, and an angiography was performed to confirm the vessel occlusion and assess the artery flow (TICI).
- Device procedure: The target site of the vessel was reached with the ANA and the CM, the clot was retrieved, and all the system was withdrawn outside the body.
- Device usability and performance: After the procedure, interventionists provided feedback on the usability and performance of the devices following the endpoints shown in Table 6. Subjective evaluation of devices usability and performance characteristics was based on the judgment and experience of the interventionist according to the rating scale. R&D Anaconda team also provided a qualitative assessment of the device's usability and performance. Recanalization was calculated using mTICI scale before clot removal and after clot removal in subclavian or carotid artery branches.
- Radial forces (RF) against the wall of the blood vessel: The radial forces were obtained using the equipment RX550 from Machine Solutions Inc. With this equipment the radial force and the diameter while decreasing and/or increasing diameter was measured according to all the stent profile. The specific geometry of the head (12 segments) of the equipment allowed the radial compression of the sample uniformly, with very low friction force. 5 cycles of compression/decompression were done in order to obtain curves defining how the radial forces vary with the diameter.

Additionally, semi-quantitative microscopic artery segments evaluation was performed by applying the following histological endpoints: endothelial denudation, intraluminal and mural thrombus, edema edemas, ruptures, hemorrhage, inflammation or injuries in internal, medial or external layers and stenosis.

### Results

The evaluation of the different Conda prototypes in combination with the guide catheter and the ANA showed a successful performance regarding to the preparative, compatibility with ancillary devices, navigability, pushability and control-manipulation of the device to the target vessel, visibility of the funnel of the ANA device and the guide catheter, device withdrawal and hemostasis, similar to the Solitaire. The CM and funnel deployment, accuracy in the target vessel and adaptability to the vessel were correct. In general, the combination of the Conda prototypes with the funnel and microcatheter was adequate; Conda prototypes with the support of the microcatheter were easily navigated to reach the target vessel.

The visual inspections showed that the integrity of the Conda prototypes, the funnel of the ANA device, the guide catheter and the Solitaire was practically unaltered after the application of 3 passes.

Table 5 summarizes the results of the procedures in renal arteries without clot and the performance and usability of the CMs during the interventions.

**Table 5. CMs scoring of performance and usability in renal arteries with no clot.**

| **Performance and usability endpoints** | **Scoring** | | | | | | |
|---|---|---|---|---|---|---|---|
| | **Conda prototypes** | | | | | | **Solitaire n=5** |
| | **ID15 n=2** | **ID16 n=2** | **ID23 n=2** | **ID24 n=1** | **ID32 n=1** | **ID33 n=1** | |
| Navigability/trackability/flexibility of the clot mobilizer / stent retriever to reach the target vessel [1 poor; 2 moderate-adequate; 3 very good] | 3 | 3 | 3 | 3 | 3 | 3 | 3 |
| Pushability of the clot mobilizer / stent retriever to the target vessel. Proximal control of the device [1 poor; 2 moderate-adequate; 3 very good] | 3 | 3 | 3 | 3 | 3 | 3 | 3 |
| Clot mobilizer / stent retriever deployment [1 no; 2 partially; 3 yes] | 3 | 3 | 3 | 3 | 3 | 3 | 3 |
| Accuracy of clot mobilizer / stent retriever deployment in the target vessel [1 no 2 yes] | 2 | 2 | 2 | 2 | 2 | 2 | 2 |
| Clot mobilizer adaptability to the vessel size and curves. Is the funnel-stent completely deployed [1 yes] or kinked [2 no]? | 1 | 1 | 1 | 1 | 1 | 1 | 1 |
| Clot mobilizer / stent retriever integrity after intervention (visual inspection) [1 poor; 2 moderate; 3 good] | 3 | 1 (1) | 1 (1) | 3 | 3 | 3 | 3 |
| | | 3 (1) | 3 (1) | | | | |

Conda prototypes with the support of the microcatheter were easily navigated to reach the target area in renal arteries; the trackability, flexibility and pushability of the clot mobilizer were assessed correctly and the stent deployment and adaptability to the vessel were correct in all cases. The deployed Conda was pushed back through the vessel until being introduced inside the expanded funnel, once it was inside the aspiration was applied. In exception of Conda ID16 and ID23 (Fig. 6 and Fig. 4, respectively), which suffered alterations in their integrity during interventions in one of the two samples, all other prototypes presented a good integrity after interventions.

Table 6 and 7 summarize the results of the procedures in subclavian and carotid artery branches with clot occlusions and the performance and usability of the CMs during the interventions. Conda ID16 (Fig. 6) and ID32/ID33 (Fig. 4-5) were not evaluated in subclavian and carotid artery branches.

**Table 6. Summary of interventions of subclavian or carotid artery branches with clot. Correct = correct performance with no incidences, success to retrieve the clot.**

| **Intervention** | **Animal** | **Target artery** | **Device** | **Nº of passes** | **TICI before intervention** | **TICI after intervention** | **Performance results** |
|---|---|---|---|---|---|---|---|
| 1. | C23 | Left Brachial | Solitaire + ANA | 1 | 0 | 3 | 1^{st} pass: correct |
| 2. | C23 | Left Brachial | Solitaire + ANA | 1 | 0 | 3 | 1^{st} pass: correct |
| 3. | C21 | Left External Carotid | ID15 + ANA | 1 | 0 | 3 | 1^{st} pass: correct |
| 4. | C21 | Left External Carotid | ID15 + ANA | 1 | 0 | 3 | 1^{st} pass: correct |
| 5. | C22 | Left External Carotid | ID15 + ANA | 1 | 0 | 3 | 1^{st} pass: correct |
| 6. | C22 | Left External Carotid | ID15 + ANA | 1 | 0 | 3 | 1^{st} pass: correct |
| 7. | C27 | Left Cervical | ID24 + ANA | 1 | 0 | 3 | 1^{st} pass: correct |
| 8. | C27 | Left Cervical | ID23 + ANA | 1 | 0 | 3 | 1^{st} pass: correct |
| 9. | C29 | Left Cervical | ID23 + ANA | 1 | 0 | 3 | 1^{st} pass: correct |

**Table 7. CMs scoring of performance and usability in subclavian and carotid artery branches with clot occlusion.**

| **Performance and usability endpoints** | **Scores** | | | | |
|---|---|---|---|---|---|
| | **Conda Prototypes** | | | | **Solitaire n=2** |
| | **ID15 n=4** | **ID23 n=2** | **ID24 n=1** | **ID33 n=1** | |
| Navigability/trackability/flexibility of the clot mobilizer / stent retriever to reach the target vessel [1 poor; 2 moderate-adequate; 3 very good] | 3 | 3 | 3 | 3 | 3 |
| Pushability of the clot mobilizer / stent retriever to the target vessel. Proximal control of the device [1 poor; 2 moderate-adequate; 3 very good] | 3 | 3 | 3 | 3 | 3 |
| Clot mobilizer / stent retriever deployment [1 no; 2 partially; 3 yes] | 3 | 3 | 3 | 3 | 3 |
| Accuracy of clot mobilizer / stent retriever deployment in the target vessel [1 no; 2 yes] | 2 | 2 | 2 | 2 | 2 |
| Clot mobilizer adaptability to the vessel size and curves. Is the funnel-stent completely deployed? [1 yes] or kinked [2 no] | 1 | 1 | 1 | 1 | 1 |
| Clot mobilizer / stent retriever integrity after intervention (visual inspection) [1 poor; 2 moderate; 3 good] | 3 | 3 | 3 | 1 | 3 |
| Clot loss during retrieval [1 no; 2 yes] | 1 | 1 | 1 | 1 | 1 |
| Recanalization success (thrombus extraction: artery flow recovery) [1, TICI 0-2a=no / 2, TICI 2b-3=yes] | 2 | 2 | 2 | 1 | 2 |
| Clot extraction [1 clot completely out the funnel; 2 clot completely inside funnel-clot mobilizer/ stent retriever; 3 clot partially inside funnel-clot mobilizer / stent retriever; 4 clot inside funnel catheter-clot mobilizer / stent retriever] | 2 | 2 | 2 | NA | 2 |

The clot extraction maneuverability was successful after the first pass in all procedures (TICI 2b-3), with no need of additional passes. The general performance and usability of the Conda prototypes were comparable to procedures in renal arteries. Solitaire performance during the clot retrieval was successful after 1^{st} pass, even though a distal embolization occurred in the left brachial artery of pig C23. Solitaire performance during the clot retrieval was successful after three passes on pig C23 but not in C25, where problems of interaction with the funnel occurred, all problems were reverted after the 2^{nd} or 3^{rd} pass.

Conda compatibility with conjunction devices resulted excellent in most of the interventions and the integrity of the clot mobilizer and funnel after intervention resulted good in most of the interventions.

In Table 8, the results of RFs and the vascular damage results on the distal segment of the vessel are presented. The results of RFs for the Trevo device (described in Example 1) were also calculated. The RF were measured at 2 mm because it is the lowest diameter for which the Conda is intended. Table 9 shows the RF values at 2 mm (average of different samples values) with a variation of ± 0.1.

**Table 8. Radial Forces (RF) of the CMs against the wall of the blood vessel.**

| **Prototype ID** | **ID15** | **ID16** | **ID23** | **ID24** | **ID32** | **ID33** | **Trevo** | **Solitaire** |
|---|---|---|---|---|---|---|---|---|
| **Average RF at 2 mm [N]** | 1.8 | 2.1 | 2.0 | 2.1 | 1.0 | 1.1 | 1.8 | 2.2 |

As is shown in Table 8, the values of RF generated by all Conda prototypes, in relation to the diameter of the vessel, resulted between the maximum limit of 2.2 N delimited by the behavior of the Solitaire (in the diameter of 2 mm) and the minimum of 1.0 N, so the results were inscribed inside the optimal range of vessel diameters (from 1.5 to 3.5 mm).

### Conclusions

This study showed that Conda in conjunction with ANA obtained better results than Solitaire in conjunction with ANA. These results imply that the Conda is an effective device for the extraction of clots, and it is the better choice than Solitaire when used together with the ANA device.

Conda also showed very good behavior in reaching the target vessel, being adapted to the vessel wall, and without problems during the retrieval. No damage was found in Conda prototypes due to their use in conjunction with ANA device, confirming that are totally compatible with ANA.

The general performance of the Conda prototypes in combination with ANA was successful and comparable to Solitaire: device preparative, navigability and pushability. The clot extraction was successful after the first pass with all the Conda prototypes in combination with ANA. The vascular injury in vessels treated with the Conda prototypes in combination with ANA after 3 passes is minimal-mild and comparable to the control device Solitaire combined with ANA.

As final conclusions, Conda prototypes obtained great results of navigability and thus, Conda is a clear option to be used in clinical trials to increase the revascularization rate during mechanical thrombectomies.

### Example 3: Radial force comparison between Conda, Solitaire and Trevo

### Introduction

This test was developed in order to compare the outward radial forces (wall apposition) generated by three stent retrievers (Conda, Solitaire and Trevo described in Example 1) depending on the lumen diameter of the vessels. After extraction of the data, graphical description (outward radial force vs. vessel diameter) comparing Solitaire, Trevo and Conda devices, was generated.

### Methods

The devices (samples) were tested for radial force (RF). The RX550 from Machine Solutions Inc. was used to measure both, the RFs and the diameter of the devices. The specific geometry of the head (12 segments) allows compressing the sample uniformly, with very low friction force. RX550 temperature was set to the defined test temperature (37±2ºC). The samples were introduced in the head and profile was started: the RX bench decreases and/or increases the diameter according to specified profile and the RFs and diameter were recorded. Pictures of the sample during the test were taken. At the end of the test, RX550 head was opened, and the sample was retrieved from the RX550 then placed within its corresponding container. Lastly, Fig. 13 depicting the individual RF vs. diameter was plotted.

The intended use for the stent retrievers is between 1.5-4.0 mm. RF safety limits were defined starting from the vessel diameter of 2.0 mm which, in Solitaire values, was at 2.2 N. This value assured that there were not endothelial damage and, therefore, it was set as the maximum value. On the other hand, the minimum value was set at 0.5 N, which was the minimum value to extract a thrombus in proper conditions, being 1.0 N the optimal value.

### Results

As is shown in Fig. 13, the values of RF generated by Conda, in relation to the diameter of the vessel, resulted between the maximum limit of 2.2 N delimited by the behavior of the Solitaire in the same diameter and the minimum of 0.5 N, being 1.0 N the optimal value not surpassed below by most of the Conda samples, so the results were inscribed inside the optimal range of vessel diameters (from 1.5 to 3.5 mm), generating a flat curve for a broad range of vessels. The curve provided by the proposed clot mobilizer device 1 (i.e., by its different samples) resulted much smoother and more constant than the curve of Solitaire and Trevo. Conda allowed lower RFs in small diameters and larger radial forces in large diameters without, therefore, leaving the limited safety window. Remarkably, due to the fact of having less radial force in small diameters (e.g., near to 2N in a diameter near to 1.5 mm), the risk of damage the vessel is reduced, in comparison with Solitaire and Trevo.

Therefore, the outward radial forces of the Conda devices are maintained for a broad range of vessels with different diameters and the risk of damaging the vessel is reduced. In conclusion, the proposed clot mobilizer device of the current invention is usable in blood vessels of different diameters.

Throughout the description and claims the word "comprise" and its variations such as "comprising" are not intended to exclude other technical features, components, steps, operations and/or groups thereof. Additional objects, advantages and features of the invention will become apparent to those skilled in the art upon examination of the description or may be learned by practice of the The foregoing description is provided primarily for exemplary purposes and should not be interpreted to limit the scope of the invention as it is set forth in the claims.

Additionally, as used herein, the singular forms "a", "an" and "the" are intended to include the plural forms as well, unless the context clearly indicates otherwise. Although the terms "first", "second". "third", "fourth" etc may be used herein to describe various features/elements (including steps), these features/elements should not be limited by these terms, unless the context indicates otherwise. These terms may be used to distinguish one feature/element from another feature/element. Thus, a first feature/element discussed below could be termed a second feature/element, and similarly, a second feature/element discussed below could be termed a first feature/element without departing from the teachings of the present invention.

Unless otherwise indicated, all numbers expressing measurements, conditions, and so forth used in the specification are to be understood as being modified in all instances by the term "about". Accordingly, unless indicated to the contrary, the numerical parameters set forth in this specification are approximations that car vary depending upon the desired properties sought to be obtained by the presently disclosed subject matter. The phrase "about" or "approximately" may be used when describing magnitude and/or position to indicate that the value and/or position described is within a reasonable expected range of values and/or positions. For example, a numeric value may have a value that is +/- 0.1% of the stated value (or range of values), +/- 1% of the stated value (or range of values), +/- 2% of the stated value (or range of values), +/-5% of the stated value (or range of values), +/- 10% of the stated value (or range of values), etc. Any numerical values given herein should also be understood to include about or approximately that value, unless the context indicates otherwise. For example, if the value "10" is disclosed, then "about 10" is also disclosed. Any numerical range recited herein is intended to include all sub-ranges subsumed therein. It is also understood that when a value is disclosed that "less than or equal to" the value, "greater than or equal to the value" and possible ranges between values are also disclosed, as appropriately understood by the skilled artisan. For example, if the value "X" is disclosed the "less than or equal to X" as well as "greater than or equal to X" (e.g., where X is a numerical value) is also disclosed. It is also understood that the throughout the application, data is provided in a number of different formats, and that this data, represents endpoints and starting points, and ranges for any combination of the data points. For example, if a particular data point "10" and a particular data point "15" are disclosed, it is understood that greater than, greater than or equal to, less than, less than or equal to, and equal to 10 and 15 are considered disclosed as well as between 10 and 15. It is also understood that each unit between two particular units are also disclosed. For example, if 10 and 15 are disclosed, then 11, 12, 13, and 14 are also disclosed.

The present disclosure and/or some other examples have been described in the above.

According to descriptions above, various alterations may be achieved.

The scope of the present invention is defined in the following set of claims.

## Claims

1. An expandable clot mobilizer device for extraction of an occlusion from a blood vessel, comprising:
a working portion (101) comprising a plurality of crowns (110, ... 110ₙ) of cells (111), each cell (111) comprising an open area bordered by two proximal cell struts (130ₙ), two distal cell struts (132ₙ), and two middle cell struts (112ₙ), the proximal cell struts (130ₙ) each extending distally from a common proximal end (134ₙ) to a proximal end (135ₙ) of a respective one of the two middle cell struts (112ₙ), the distal cell struts (132ₙ) each extending proximally from a common distal end (136ₙ₊₁) to a distal end (133ₙ) of a respective one of the two middle cell struts (112ₙ), each middle cell strut (112) in each crown bordering two adjacent cells (111) in that crown, each of the middle cell struts (112) being adapted and configured to be more flexible than the distal cell strut (132) and the proximal cell strut (130) to which it extends; and
a tapered portion (120) extending proximally from a proximal end of the working portion (101), the tapered portion (120) comprising a plurality of tapered portion struts (122), the tapered portion (120) having a smaller diameter at a proximal end than a diameter of the working portion (101) in an expanded configuration,
**characterized in that**:
the working portion (101) further comprises at least one crown (115) of intermediate cells (116), each intermediate cell (116) comprising an open area bordered by two proximal cell struts (108) intersecting with two distal cell struts (109).

2. The device of claim 1, wherein the working portion (101) defines a tubular-shaped section forming a cylindrically closed structure.

3. The device of any of claims 1 or 2, wherein the middle cell struts (112) bordering each cell (111) have a width less than a width of the distal cell struts (132) bordering that cell and less than a width of the proximal cell struts (130) bordering that cell (111).

4. The device of any of the previous claims, wherein the distal cell struts (132) each have a width greater than or equal to 0.10 mm and less than or equal to 0.145 mm, the proximal cell struts (130) each have a width greater than or equal to 0.10 mm and less than or equal to 0.145 mm, and the middle cell struts (112) each have a width greater than or equal to 0.05 mm and less than or equal to 0.10 mm.

5. The device of any of the previous claims, wherein the working portion (101) comprises at least four crowns (110₁, 110₂, 110₃, 110₄) of cells (111) and each crown (110ₙ) of cells (111) comprises at least four cells (111).

6. The device of any of the previous claims, wherein the middle cell strut (112) comprises at least a hinge portion (140) disposed between the proximal (135) and the distal (133) ends of the middle cell strut (112).

7. The device of any of the previous claims, wherein in each crown of cells:
the distal cell struts (132ₙ) are disposed in a distal cell strut ring (114ₙ) at equal distal cell strut positions along a longitudinal axis of the clot mobilizer device (1),
the proximal cell struts (130ₙ) are disposed in a proximal cell strut ring (113ₙ) at equal proximal cell strut positions along the longitudinal axis of the clot mobilizer device (1), and
the common proximal ends (134ₙ₊₁) of the distal cell strut ring (114ₙ) are circumferentially offset from the common distal ends (136ₙ) of the proximal cell strut ring (113ₙ).

8. The device of claim 7, wherein the common proximal ends (134ₙ₊₁) of the distal cell strut ring (114ₙ) of each crown (110ₙ) of cells (111) are circumferentially offset from the common distal ends (136ₙ) of the proximal cell strut ring (113ₙ) of that crown (110ₙ) of cells (111) in one circumferential direction.

9. The device of claim 7, wherein the common proximal ends (134₂) of the distal cell strut ring (114₁) of a first crown (110₁) of cells (111) are offset from the common distal ends (136₁) of the proximal cell strut ring (113₁) of the first crown (110₁) of cells (111) in a first circumferential direction and the common proximal ends (134₃) of the distal cell strut ring (114₂) of a second crown (110₂) of cells (111) are offset from the common distal ends (136₂) of the proximal cell strut ring (113₂) of the second crown (110₂) of cells (111) in a second circumferential direction opposite to the first circumferential direction.

10. The device of any of the previous claims, wherein the middle cell struts (112₂) of a second crown (110₂) of cells (111) extend distally from common distal ends (136₂) of distal cell struts (114₁) of a first crown (110₁) of cells (111) to common proximal ends (134₃) of proximal cell struts (113₃) of a third crown (110₃) of cells (111), and the middle cell struts (112₃) of the third crown (110₃) of cells (111) extend distally from common distal ends (136₃) of distal cell struts (114₂) of the second crown (110₂) of cells (111) to common proximal ends (134₄) of proximal cell struts (113₄) of a fourth crown (110₄) of cells (111).

11. The device of claim 1, wherein the distal cell struts (109) of each intermediate cell (116) in the at least one crown (115) of intermediate cells (116) comprise the proximal cell struts (130) of cells (111) in an adjacent crown (110) of cells (111), and the proximal cell struts (108) of each intermediate cell (116) in the at least one crown (115) of intermediate cells (116) comprise the distal cell struts (132) of cells (111) in an adjacent crown of cells (110).

12. The device of any of the previous claims, wherein the working portion (101) is configured to expand from a compressed configuration with a first diameter of less than 1.5 mm to an expanded configuration with a second diameter of at least 3.0 mm and to exert an outward radial force between 0.5 N and 3 N at every diameter between and including the first diameter and the second diameter.

13. The device of any of the previous claims, wherein at least some of the plurality of tapered portion struts (122) border a plurality of tapered portion cells (124), wherein at least some of the plurality of tapered portion struts (122) converge from a proximal end of the working portion (101) to a distal end of a proximal connection portion (121), the clot mobilizer device (1) further comprising a pusher (200) extending proximally from the proximal connection portion (121).

14. The device of claim 13, wherein:
the proximal connection portion (121) has a first attachment surface (102);
the pusher (200) comprises a second connection portion (201) extending distally, the second connection portion (201) having a second attachment surface (202) facing and extending longitudinally aligned to the first attachment surface (102) to define an overlapping portion and to define first and second seams extending longitudinally along first and second lateral extents of the overlapping portion;
the clot mobilizer device (1) further comprising:
a first weld attaching the proximal and second connection portions (121, 201), extending from the first seam toward the second seam; and
a second weld attaching the proximal and second connection portions (121, 201), extending from the second seam toward the first seam.

## Patentansprüche

1. Expandierbare Thrombus-Mobilisierungsvorrichtung zur Entfernung eines Gefäßverschlusses, umfassend:
einen Funktionsteil (101) mit einer Vielzahl von Kronen (110₁ ... 110ₙ) aus Zellen (111), wobei jede Zelle (111) einen offenen Bereich aufweist, der durch zwei proximale Zellstreben (130ₙ), zwei distale Zellstreben (132ₙ) und zwei mittlere Zellstreben (112ₙ) begrenzt ist, wobei sich die proximalen Zellstreben (130ₙ) jeweils distal von einem gemeinsamen proximalen Ende(134ₙ) bis zu dem proximalen Ende (135ₙ) der jeweils zugeordneten der beiden mittleren Zellstreben (112ₙ) erstrecken, wobei sich die distalen Zellstreben (132ₙ) jeweils proximal von einem gemeinsamen distalen Ende (136ₙ₊₁) bis zu dem distalen Ende (133ₙ) der jeweils zugeordneten der beiden mittleren Zellstreben (112ₙ) erstrecken, wobei jede mittlere Zellstrebe (112) in jeder Krone an zwei benachbarte Zellen (111) in dieser Krone angrenzt, wobei jede der mittleren Zellstreben (112) so ausgebildet und ausgelegt ist, dass sie flexibler ist als die distale Zellstrebe (132) und die proximale Zellstrebe (130), zu denen sie sich erstreckt; und
einen Verjüngungsteil (120), der sich proximal vom proximalen Ende des Funktionsteils (101) erstreckt, wobei der Verjüngungsteil (120) eine Vielzahl von Verjüngungsteilstreben (122) umfasst, wobei der Verjüngungsteil (120) an seinem proximalen Ende einen geringeren Durchmesser aufweist als der Funktionsteil (101) in expandierter Anordnung,
**dadurch gekennzeichnet, dass**:
der Funktionsteil (101) ferner mindestens eine Krone (115) aus Zwischenzellen (116) umfasst, wobei jede Zwischenzelle (116) einen offenen Bereich aufweist, der von zwei proximalen Zellstreben (108), die zwei distale Zellstreben (109) kreuzen, begrenzt wird.

2. Vorrichtung nach Anspruch 1, wobei der Arbeitsabschnitt (101) einen röhrenförmigen Abschnitt in Form einer zylindrisch geschlossenen Struktur aufweist.

3. Vorrichtung nach einem der Ansprüche 1 oder 2, wobei die Breite der mittleren Zellstreben (112), die die einzelnen Zellen (111) begrenzen, geringer ist als die Breite der distalen Zellstreben (132), die diese Zelle begrenzen, und geringer als die Breite der proximalen Zellstreben (130), die diese Zelle (111) begrenzen.

4. Vorrichtung nach einem der vorstehenden Ansprüche, wobei die distalen Zellstreben (132) jeweils eine Breite größer oder gleich 0,10 mm und kleiner oder gleich 0,145 mm aufweisen, die proximalen Zellstreben (130) jeweils eine Breite größer oder gleich 0,10 mm und kleiner oder gleich 0,145 mm aufweisen, und die mittleren Zellstreben (112) jeweils eine Breite größer oder gleich 0,05 mm und kleiner oder gleich 0,10 mm aufweisen.

5. Vorrichtung nach einem der vorstehenden Ansprüche, wobei der Funktionsteil (101) mindestens vier Kronen (110₁, 110₂, 110₃, 110₄) aus Zellen (111) umfasst und jede Krone (110ₙ) aus Zellen (111) mindestens vier Zellen (111) umfasst.

6. Vorrichtung nach einem der vorstehenden Ansprüche, wobei die mittlere Zellstrebe (112) mindestens einen dem proximalen (135) und dem distalen (133) Ende der mittleren Zellstrebe (112) angeordneten Gelenkabschnitt (140) umfasst.

7. Vorrichtung nach einem der vorstehenden Ansprüche, wobei in jeder Krone aus Zellen:
die distalen Zellstreben (132ₙ) in einem Distalzellstrebenring (114ₙ) an gleichen Distalzellstrebenpositionen entlang der Längsachse der Thrombus-Mobilisierungsvorrichtung (1) angeordnet sind,
die proximalen Zellstreben (130ₙ) in einem Proximalzellstrebenring (113ₙ) an gleichen Proximalzellstrebenpositionen entlang der Längsachse der Thrombus-Mobilisierungsvorrichtung (1) angeordnet sind, und
die gemeinsamen proximalen Enden (134ₙ₊₁) des Distalzellstrebenrings (114₁) zu den gemeinsamen distalen Enden(136ₙ) des Proximalzellstrebenrings (113ₙ) zirkumferenziell versetzt sind.

8. Vorrichtung nach Anspruch 7, wobei die gemeinsamen proximalen Enden (134ₙ₊₁) des Distalzellstrebenrings (114ₙ) jeder Krone (110ₙ) aus Zellen (111) zu den gemeinsamen distalen Enden (136ₙ) des Proximalzellstrebenrings (113ₙ) derselben Krone (110ₙ) aus Zellen (111) in ein und derselben Umfangsrichtung zirkumferenziell versetzt sind.

9. Vorrichtung nach Anspruch 7, wobei die gemeinsamen proximalen Enden (134₂) des Distalzellstrebenrings(114₁) einer ersten Krone (110₁) aus Zellen (111) zu den gemeinsamen distalen Enden (136₁) des Proximalzellstrebenrings (113₁) der ersten Krone (110₁) aus Zellen (111) in einer ersten Umfangsrichtung versetzt sind und die gemeinsamen proximalen Enden (134₃) des Distalzellstrebenrings (114₂) einer zweiten Krone (110₂) aus Zellen (111) zu den gemeinsamen distalen Enden(136₂) des Proximalzellstrebenrings (113₂) der zweiten Krone (110₂) aus Zellen (111) in einer zweiten Umfangsrichtung entgegengesetzt zur ersten Umfangsrichtung versetzt sind.

10. Vorrichtung nach einem der vorstehenden Ansprüche, wobei sich die mittleren Zellstreben (112₂) einer zweiten Krone (110₂) aus Zellen (111) distal von gemeinsamen distalen Enden (136₂) distaler Zellstreben (114₁) einer ersten Krone (110₁) aus Zellen (111) bis zu gemeinsamen proximalen Enden (134₃) proximaler Zellstreben(113₃) einer dritten Krone (110₃) aus Zellen (111) erstrecken, und die mittleren Zellstreben (112₃) der dritten Krone (110₃) aus Zellen (111) sich distal von gemeinsamen distalen Enden (136₃) distaler Zellstreben (114₂) der zweiten Krone (110₂) aus Zellen (111) bis zu gemeinsamen proximalen Enden (134₄) proximaler Zellstreben (113₄) einer vierten Krone (110₄) aus Zellen (111) erstrecken.

11. Vorrichtung nach Anspruch 1, wobei die distalen Zellstreben (109) jeder Zwischenzelle (116) in der mindestens einen Krone (115) aus Zwischenzellen (116) die proximalen Zellstreben (130) von Zellen (111) in einer benachbarten Krone (110) aus Zellen (111) umfassen, und wobei die proximalen Zellstreben (108) jeder Zwischenzelle (116) in der mindestens einen Krone (115) aus Zwischenzellen (116) die distalen Zellstreben (132) von Zellen (111) in einer benachbarten Krone aus Zellen (110) umfassen.

12. Vorrichtung nach einem der vorstehenden Ansprüche, wobei der Funktionsteil (101) so ausgelegt ist, dass er sich aus einer komprimierten Anordnung mit einem ersten Durchmesser von weniger als 1,5 mm in eine expandierte Anordnung mit einem zweiten Durchmesser von mindestens 3,0 mm ausdehnt und dabei bei jedem Durchmesser zwischen dem ersten Durchmesser und dem zweiten Durchmesser, einschließlich beider, eine nach außen gerichtete Radialkraft zwischen 0,5 N und 3 N ausübt.

13. Vorrichtung nach einem der vorstehenden Ansprüche, wobei mindestens einige aus der Vielzahl von Verjüngungsteilstreben (122) eine Vielzahl von Verjüngungsteilzellen (124) begrenzen, wobei mindestens einige aus der Vielzahl von Verjüngungsteilstreben (122) vom proximalen Ende des Funktionsteils (101) zum distalen Ende eines proximalen Verbindungsteils (121) konvergieren, wobei die Thrombus-Mobilisierungsvorrichtung (1) ferner ein Schubelement (200) umfasst, das sich proximal an den proximalen Verbindungsteil (121) anschließt.

14. Vorrichtung nach Anspruch 13, wobei:
der proximale Verbindungsteil (121) eine erste Befestigungsfläche (102) aufweist;
das Schubelement (200) einen zweiten Verbindungsteil (201) umfasst, der sich in distaler Richtung erstreckt und eine zweite Befestigungsfläche (202) aufweist, die der ersten Befestigungsfläche (102) zugewandt und in Längsrichtung fluchtend angeordnet ist, unter Bildung eines Überlappungsbereichs sowie unter Ausbildung einer ersten und einer zweiten Naht, die sich jeweils entlang einer ersten bzw. zweiten seitlichen Ausdehnung des Überlappungsbereichs in Längsrichtung erstrecken;
wobei die Thrombus-Mobilisierungsvorrichtung (1) ferner Folgendes umfasst:
eine erste Verschweißung, die den proximalen und den zweiten Verbindungsteil (121, 201) verbindet und sich von der ersten Naht in Richtung der zweiten Naht erstreckt; und
eine zweite Verschweißung, die den proximalen und den zweiten Verbindungsteil (121, 201) verbindet und sich von der zweiten Naht in Richtung der ersten Naht erstreckt.

## Revendications

1. Un dispositif expansible de mobilisation de caillot pour l'extraction d'une occlusion d'un vaisseau sanguin, comprenant :
une partie active (101) comprenant une pluralité de couronnes (110, ... 110ₙ) de cellules (111), chaque cellule (111) comprenant une zone ouverte délimitée par deux montants proximaux de cellule (130n), deux montants distaux de cellule (132n) et deux montants intermédiaires de cellule (112n), les montants proximaux de cellule (130n) s'étendant chacun distalement depuis une extrémité proximale commune (134n) jusqu'à une extrémité proximale (135n) d'un des deux montants intermédiaires de cellule (112n) respectifs, les montants distaux de cellule (132n) s'étendant chacun proximalement depuis une extrémité distale commune (136n+1) jusqu'à une extrémité distale (133n) d'un des deux montants intermédiaires de cellule (112n) respectifs, chaque montant intermédiaire de cellule (112) dans chaque couronne bordant deux cellules adjacentes (111) dans cette couronne, chacun des montants intermédiaires de cellule (112) étant conçu et configuré pour être plus flexible que le montant distal de cellule (132) et le montant proximal de cellule (130) auxquels il est relié ;
et une portion conique (120) s'étendant proximalement à partir d'une extrémité proximale de la partie active (101), la portion conique (120) comprenant une pluralité de montants de portion conique (122), la portion conique (120) ayant un diamètre plus petit à son extrémité proximale qu'un diamètre de la partie active (101) dans une configuration déployée, **caractérisé en ce que** :
la partie active (101) comprend en outre au moins une couronne (115) de cellules intermédiaires (116), chaque cellule intermédiaire (116) comprenant une zone ouverte délimitée par deux montants proximaux de cellule (108) croisant deux montants distaux de cellule (109).

2. Le dispositif selon la revendication 1, dans lequel la partie active (101) définit une section en forme de tube formant une structure cylindriquement fermée.

3. Le dispositif selon l'une quelconque des revendications 1 ou 2, dans lequel les montants intermédiaires de cellule (112) bordant chaque cellule (111) ont une largeur inférieure à celle des montants distaux de cellule (132) bordant cette cellule et inférieure à celle des montants proximaux de cellule (130) bordant cette cellule (111).

4. Le dispositif selon l'une quelconque des revendications précédentes, dans lequel les montants distaux de cellule (132) ont chacun une largeur supérieure ou égale à 0,10 mm et inférieure ou égale à 0,145 mm, les montants proximaux de cellule (130) ont chacun une largeur supérieure ou égale à 0,10 mm et inférieure ou égale à 0,145 mm, et les montants intermédiaires de cellule (112) ont chacun une largeur supérieure ou égale à 0,05 mm et inférieure ou égale à 0,10 mm.

5. Le dispositif selon l'une quelconque des revendications précédentes, dans lequel la partie active (101) comprend au moins quatre couronnes (110₁, 110₂, 110₃, 110₄) de cellules (111) et chaque couronne (110ₙ) de cellules (111) comprend au moins quatre cellules (111).

6. Le dispositif selon l'une quelconque des revendications précédentes, dans lequel le montant intermédiaire de cellule (112) comprend au moins une portion articulée (140) disposée entre les extrémités proximale (135) et distale (133) du montant intermédiaire de cellule (112).

7. Le dispositif selon l'une quelconque des revendications précédentes, dans lequel, dans chaque couronne de cellules :
les montants distaux de cellule (132ₙ) sont disposés dans un anneau de montants distaux de cellule (114ₙ) à des positions équidistantes le long d'un axe longitudinal du dispositif de mobilisation de caillot (1),
les montants proximaux de cellule (130ₙ) sont disposés dans un anneau de montants proximaux de cellule (113ₙ) à des positions équidistantes le long de l'axe longitudinal du dispositif de mobilisation de caillot (1), et
les extrémités proximales communes (134ₙ₊₁) de l'anneau de montants distaux de cellule (114ₙ) sont décalées en circonférence par rapport aux extrémités distales communes (136ₙ) de l'anneau de montants proximaux de cellule (113ₙ).

8. Le dispositif selon la revendication 7, dans lequel les extrémités proximales communes (134ₙ₊₁) de l'anneau de montants distaux de cellule (114ₙ) de chaque couronne (110ₙ) de cellules (111) sont décalées en circonférence par rapport aux extrémités distales communes (136ₙ) de l'anneau de montants proximaux de cellule (113ₙ) de cette couronne (110ₙ) de cellules (111) dans une direction circonférentielle.

9. Le dispositif selon la revendication 7, dans lequel les extrémités proximales communes (134₂) de l'anneau de montants distaux de cellule (114₁) d'une première couronne (110₁) de cellules (111) sont décalées par rapport aux extrémités distales communes (136₁) de l'anneau de montants proximaux de cellule (113₁) de la première couronne (110₁) de cellules (111) dans une première direction circonférentielle, et les extrémités proximales communes (134₃) de l'anneau de montants distaux de cellule (114₂) d'une seconde couronne (110₂) de cellules (111) sont décalées par rapport aux extrémités distales communes (136₂) de l'anneau de montants proximaux de cellule (113₂) de la seconde couronne (110₂) de cellules (111) dans une seconde direction circonférentielle opposée à la première direction circonférentielle.

10. Le dispositif selon l'une quelconque des revendications précédentes, dans lequel les montants intermédiaires de cellule (112₂) d'une seconde couronne (110₂) de cellules (111) s'étendent distalement depuis les extrémités distales communes (136₂) des montants distaux de cellule (114₁) d'une première couronne (110₁) de cellules (111) jusqu'aux extrémités proximales communes (134₃) des montants proximaux de cellule (113₃) d'une troisième couronne (110₃) de cellules (111), et les montants intermédiaires de cellule (112₃) de la troisième couronne (110₄) de cellules (111) s'étendent distalement depuis les extrémités distales communes (136₃) des montants distaux de cellule (114₂) de la seconde couronne (110₂) de cellules (111) jusqu'aux extrémités proximales communes (134₄) des montants proximaux de cellule (113₄) d'une quatrième couronne (110₄) de cellules (111).

11. Le dispositif selon la revendication 1, dans lequel les montants distaux de cellule (109) de chaque cellule intermédiaire (116) dans ladite au moins une couronne (115) de cellules intermédiaires (116) comprennent les montants proximaux de cellule (130) de cellules (111) dans une couronne adjacente (110) de cellules (111), et les montants proximaux de cellule (108) de chaque cellule intermédiaire (116) dans ladite au moins une couronne (115) de cellules intermédiaires (116) comprennent les montants distaux de cellule (132) de cellules (111) dans une couronne adjacente de cellules (110).

12. Le dispositif selon l'une quelconque des revendications précédentes, dans lequel la partie active (101) est configurée pour passer d'une configuration comprimée avec un premier diamètre inférieur à 1,5 mm à une configuration déployée avec un second diamètre supérieur ou égal à 3,0 mm, et pour exercer une force radiale dirigée vers l'extérieur comprise entre 0,5 N et 3 N pour chaque diamètre compris entre et incluant le premier et le second diamètre.

13. Le dispositif selon l'une quelconque des revendications précédentes, dans lequel au moins certains des montants (122) de la portion conique délimitent une pluralité de cellules (124) de portion conique, au moins certains desdits montants (122) de la portion conique convergeant depuis une extrémité proximale de la partie active (101) vers une extrémité distale d'une portion de connexion proximale (121), le dispositif de mobilisation de caillot (1) comprenant en outre un poussoir (200) s'étendant proximalement à partir de ladite portion de connexion proximale (121).

14. Le dispositif selon la revendication 13, dans lequel :
la portion de connexion proximale (121) comporte une première surface de fixation (102) ;
le poussoir (200) comprend une seconde portion de connexion (201) s'étendant distalement, la seconde portion de connexion (201) comportant une seconde surface de fixation (202) faisant face à la première surface de fixation (102) et s'étendant de manière longitudinalement alignée à celle-ci de façon à définir une portion de recouvrement, ainsi que des première et seconde lignes de jonction s'étendant longitudinalement le long des première et seconde limites latérales de la portion de recouvrement ;
le dispositif de mobilisation de caillot (1) comprenant en outre :
une première soudure reliant les portions de connexion proximale et seconde (121, 201), s'étendant depuis la première ligne de jonction vers la seconde ligne de jonction ; et
une seconde soudure reliant lesdites portions de connexion (121, 201), s'étendant depuis la seconde ligne de jonction vers la première ligne de jonction.
